# FASCICULE DE BREVET EUROPEEN

(11) **EP 2 342 247 B1**
(45) Date de publication et mention de la délivrance du brevet: **27.01.2016**
(21) Numéro de dépôt: 09737043.1
(22) Date de dépôt: 01.09.2009
(51) Int. Cl.: C08F 293/00, C08F 220/00, C08F 2/48, C08F 2/38, C07C 333/20, C07D 249/04, B01J 20/26, C08F 222/10, C08F 8/14, C08F 8/34, C08F 8/30

(54) **PROCÉDÉ DE PRÉPARATION DE POLYMÈRES À EMPREINTES MOLÉCULAIRES (PEM) PAR POLYMÉRISATION RADICALAIRE**
VERFAHREN ZUR HERSTELLUNG VON MOLEKULAR GEPRÄGTEN POLYMEREN (PEM) DURCH RADIKALPOLYMERISATION
METHOD FOR PREPARING MOLECULAR IMPRINT POLYMERS (PEM) BY RADICAL POLYMERISATION

(30) Priorité: 05.09.2008 FR 0804874
(43) Date de publication de la demande: 13.07.2011
(73) Titulaire: Université de Technologie de Compiègne - UTC, 60205 Compiègne Cedex (FR)
(72) Inventeur: HAUPT, Karsten, F-60190 Pronleroy (FR); CUTIVET, Arnaud, F-69100 Villeurbanne (FR); TSE SUM BUI, Jeanne Bernadette, F-60190 Pronleroy (FR); ÇAKIR, Pinar, F-60200 Compiègne (FR)
(74) Mandataire: Novagraaf Technologies
(86) Numéro de dépôt international: PCT/FR2009/001051
(87) Numéro de publication internationale: WO 2010/026308

(56) Documents cités:
- EP-A- 1 767 551
- US-B1- 6 379 599
- US-B1- 6 525 154

## Description

### Domaine technique

La présente invention se rapporte à un procédé de préparation d'un polymère à empreinte(s) moléculaire(s) (PEM) par polymérisation radicalaire mettant en oeuvre au moins un amorceur comportant au moins deux fonctions chimiques capables de former des radicaux réactifs, et de ce fait obtenir une concentration localement élevée de radicaux permettant d'amorcer la polymérisation sur ou autour de l'entité ou molécule empreinte.

Elle concerne également les polymères à empreinte(s) moléculaire(s) (PEM) susceptibles d'être obtenus par le procédé de l'invention ainsi que leur utilisation dans la réalisation des biocapteurs biomimétiques, des biopuces biomimétiques, des capteurs chimiques, des dispositifs de séparation par adsorption spécifique, en tant que revêtement notamment pour le relargage de produits actifs.

Dans la description ci-dessous, les références entre crochets ([ ]) renvoient à la liste des références présentée à la fin du texte.

### Etat de la technique

Les capteurs chimiques ou les biocapteurs sont des dispositifs permettant de retenir au moins temporairement une substance chimique ou biologique à détecter et/ou à quantifier comme par exemple la mesure ou détection d'un gaz (mesure de la pollution, de réactifs chimiques, etc.), la détection d'un principe actif (médicament, herbicide, etc.), la détection de substances humaines ou animales (cellules, protéines, etc.), des virus, etc.

Les biocapteurs et les phases stationnaires de séparation par affinité actuellement disponibles utilisent souvent des biomacromolécules ou macromolécules biologiques comme éléments de reconnaissance spécifiques, notamment des anticorps, des enzymes ou des acides nucléiques. Lesdites biomacromolécules présentent une faible stabilité chimique et physique, notamment aux solvants organiques, aux acides et bases, aux températures élevées. Les biomacromolécules sont coûteuses et difficiles à obtenir, et difficiles à intégrer dans des processus de fabrication industriels. Dans ce contexte, l'utilisation de polymères et/ou de copolymères présentant un ou plusieurs types d'empreinte(s) moléculaire(s) (PEM) s'impose comme une alternative à ces biomacromolécules. Ce sont des matériaux biomimétiques qui reproduisent la capacité de reconnaissance moléculaire que l'on observe chez certaines macromolécules biologiques [1].

Les polymères présentant un ou plusieurs types d'empreinte(s) moléculaire(s) (PEM) existent déjà. Toutefois, leur utilisation pratique se trouve freinée par une mise en oeuvre trop complexe sous la forme optimale pour l'application concernée.

La synthèse de polymères par polymérisation radicalaire utilisant des concentrations relativement faibles d'amorceur et de monomères, comme dans le cas de l'amorceur immobilisé sur un support solide [2] ou en solution diluée pour la synthèse de microgels et nanogels [3] a déjà été décrite. Le polymère obtenu présente en général une forme de "fractale", pas assez dense pour son adaptation à la synthèse de PEM [4].

Pour la synthèse de polymères PEM sous forme de microgels, il a été proposé de pallier à cet inconvénient en utilisant une concentration de réactifs élevée dans un premier temps, pour diluer la solution juste avant la macrogélation afin d'obtenir des microgels denses [5]. Cette approche est pourtant délicate et difficile à mettre en oeuvre surtout dans un contexte industriel, du fait qu'une augmentation d'échelle paraît difficile à réaliser de façon reproductible.

Il existe donc un réel besoin d'un procédé de préparation de polymères à empreinte(s) moléculaire(s) (PEM) palliant ces défauts, inconvénients et obstacles de l'art antérieur.

En particulier, il existe un réel besoin d'un procédé qui soit reproductible, industriellement réalisable, qui permette la préparation de polymères à empreinte(s) moléculaire(s) (PEM) de façon dense et localisée, à l'échelle micro ou nanométrique, qui, en outre, puisse permettre un contrôle de la taille, de la morphologie et de la forme des polymères (PEM) obtenus.

### Description de l'invention

La présente invention a précisément pour but de répondre à ce besoin en fournissant un procédé de préparation d'un polymère à empreinte(s) moléculaire(s) (PEM) par polymérisation radicalaire dans lequel :
a) on met en contact :
   ▪ au moins une entité ou molécule empreinte ;
   ▪ des monomères identiques ou différents, capables de former le polymère à empreinte(s) moléculaire(s) (PEM) ;
   ▪ au moins un amorceur comportant au moins deux fonctions chimiques (F1) capables de former des radicaux permettant d'amorcer la polymérisation desdits monomères sur ou autour de ladite entité ou molécule empreinte ;
b) on effectue la polymérisation sous l'action d'un rayonnement lumineux de longueur d'onde visible ou UV ou sous l'action d'un apport d'énergie thermique formant ainsi sur ou autour de ladite entité ou molécule empreinte un polymère à empreinte(s) moléculaire(s) (PEM) ;
c) on élimine le ou les monomères non polymérisés et/ou tout ou partie de l'entité ou molécule empreinte du polymère formé en b) avec un solvant ou un mélange de solvant(s) approprié(s).

Le procédé de polymérisation selon l'invention met en oeuvre un nouveau type d'amorceur pour la préparation de polymères à empreinte(s) moléculaire(s) (PEM) qui permet la génération localisée d'un nombre élevé de radicaux et une bonne maîtrise de la taille, de la forme et de la morphologie des chaînes macromoléculaires pendant la polymérisation.

Ceci permet d'améliorer les propriétés des polymères (PEM) et ainsi d'optimiser à la fois la sélectivité de la reconnaissance et la sensibilité de la réponse d'un dispositif basé sur un tel polymère (PEM).

L'obtention du polymère à empreinte(s) moléculaire(s) (PEM) repose sur le principe de l'impression moléculaire. L'impression moléculaire est une technique bien connue de l'homme du métier. Elle consiste globalement dans son principe fondamental à créer des images complémentaires d'une molécule dans un matériau, en général un polymère synthétique. La ou les molécules ou entités contre lesquelles des empreintes doivent être générées (molécule ou entité empreinte au sens de la présente invention) sont mises en contact avec un mélange de monomères portant des groupements fonctionnels capables de complexer la ou lesdites molécules ou entités par liaisons non-covalentes, par liaisons de coordination ou par liaisons covalentes réversibles.

Comme il sera expliqué plus en détail ci-après, il est essentiel que la nature de la liaison de chaque entité ou molécule empreinte avec le PEM soit réversible de sorte à pouvoir, dans des conditions déterminées, détacher ou délier ladite ou lesdites entités ou molécules du polymère ou matériaux à empreinte(s) que l'on souhaite synthétiser et qui servira ensuite à détecter, capter et/ou relarguer les substances pertinentes en fonction de l'application ou de l'usage souhaité.

Dans la description les termes polymère et matériau à empreinte(s) moléculaire(s) peuvent être utilisés indifféremment. Par polymère ou matériau, au sens de la présente invention, il faut comprendre tous les types de polymères, d'homopolymères (synthétisé à partir d'un seul type de monomère et dont tous les motifs de répétition sont de même nature chimique) et de copolymères (un polymère contenant plusieurs types de motifs de répétition) organiques ou inorganiques habituellement mis en oeuvre pour ce genre de technique.

Au sens de l'invention, un radical est une espèce chimique possédant un ou plusieurs électrons non appariés. La présence d'un électron célibataire confère à ces espèces une grande réactivité, ce qui signifie qu'elles ont la possibilité de réagir avec d'autres composés.

Par ailleurs, dans la suite de la description les termes « se fixer » et « se lier » sont indifféremment utilisés pour signifier la liaison qui se forme entre les atomes ou molécules. Selon le cas, il peut s'agir de liaison : covalente, de coordination, hydrogène, ionique, Van der Waals, ou d'une interaction hydrophobe. Selon les cas, la liaison peut être réversible ou non.

Dans le cadre de la présente invention, par dendron on entend une molécule arborescente construite par des processus itératifs (pouvant se produire plusieurs fois) à partir d'une molécule comportant, d'une part, un site ayant des branchements munis d'extrémités réactives et, d'autre part, un site apte à se fixer sur un support moléculaire ou solide. La taille du dendron est fonction du nombre d'itérations appelé également génération. Plus le nombre d'itérations ou générations est important, plus grande sera la taille de dendron. Une génération x correspond à x itérations, x étant un nombre entier correspondant au nombre d'itérations. Plus la génération de dendron sera importante, plus le nombre d'extrémités réactives sera important.

Par dendritique on entend une structure de type dendron. Le terme iniferter (en anglais « Initiator - Transfer Agent - Terminator » et en français « Agent Initiateur, de Transfert et de Terminaison ») désigne un type d'amorceur de polymérisation radicalaire vivante tel que décrit dans la référence [6].

Comme indiqué, la polymérisation des monomères peut se faire sur l'entité ou molécule empreinte ou autour de celle-ci.

La polymérisation se fait sur l'entité ou molécule empreinte lorsque le ou les polymères formés sont liés directement à ladite entité ou molécule et ne l'enrobent que partiellement. Cela peut se produire en particulier lorsque l'entité ou molécule empreinte et le ou les polymères formés sont de dimensions voisines. Cela peut s'observer par exemple si un polymère (PEM) sous forme de particules de taille nanométrique est synthétisé pour une protéine comme molécule empreinte qui elle-même a un diamètre de quelques nanomètres. Cela peut se produire également lorsque l'entité ou molécule empreinte est fixée sur un support pendant la synthèse du polymère (PEM) et donc est d'une accessibilité restreinte.

La polymérisation se fait autour de l'entité ou molécule empreinte lorsque le ou les polymères formés sont liées directement à ladite entité ou molécule et l'enrobent complètement à la fin de la polymérisation. Un exemple est un polymère (PEM) sous forme de nanoparticules pour une petite molécule empreinte comme un aminoacide ; dans ce cas le polymère (PEM) a une taille plus grande d'un ou plusieurs ordres de grandeur que la molécule empreinte.

Dans l'étape a), l'entité ou molécule empreinte est destinée à générer dans le polymère des empreintes moléculaires, le résultat étant un polymère à empreinte(s) moléculaire(s) (PEM). Au sens de l'invention, on appelle l'entité ou molécule cible, l'entité ou molécule destinée à être liée de manière réversible au polymère à empreinte(s) moléculaire(s) (PEM) lors de son utilisation.

L'entité ou molécule empreinte peut être identique à l'entité ou molécule cible. Elle peut également être un dérivé de structure et de forme proches de l'entité ou molécule cible. L'entité ou molécule empreinte peut encore correspondre à une partie seulement de l'entité ou molécule cible.

Tout type d'entité ou de molécule empreinte peut être utilisé dans le procédé de l'invention.

Plus particulièrement, l'entité ou molécule empreinte peut être choisie dans le groupe comprenant:
- les aminoacides, les oses, les lipides, les nucléosides, les nucléotides, les oligomères et polymères obtenus à partir de ces entités ou molécules, notamment les peptides, protéines, acides nucléiques, oligosaccharides et polysaccharides,
- les entités ou molécules biologiquement actives, notamment les médicaments, notamment les antibiotiques, les hormones stéroïdes, les vitamines, les toxines, les enzymes, les agents de dopage, les pesticides, insecticides, fongicides et herbicides,
- les substances explosives,
- les substances toxiques,
- les perturbateurs endocriniens,
- des mycotoxines ou toxines bactériens,
- les nanoparticules,
- les cellules de bactéries procaryotes et d'eucaryotes
- les cellules animales, notamment les cellules de levures, les cellules de champignons et les cellules animales et humaines
- les cellules de plantes,
- les virus, et
- les tissus cellulaires.

L'entité ou molécule empreinte peut être plus particulièrement choisie dans le groupe comprenant la trypsine, la kallicréine, le S-propranolol, la théophylline, l'acide 2,4-dichlorophénoxyacétique, le beta-estradiol, le testostérone, l'atrazine, la morphine, la cocaïne, le tétrahydrocannabinol.

Dans le cadre de la présente invention, il est possible de fabriquer des matériaux ne présentant qu'un seul et unique type d'empreinte (destiné à ne fixer qu'une seule entité ou molécule empreinte, de même qu'on peut prévoir plusieurs types différents d'empreintes (par exemple pour une série de molécules de nature ou de taille similaire ou ayant un même type de fonctionnalité chimique ou de récepteur biologique), ce quelque soit la densité de l'empreinte ou des différents types d'empreintes présente à la surface et ou dans le matériau (cage, réseau tridimensionnel), c'est-à-dire le nombre d'empreintes par m² ou m³.

On peut naturellement faire varier la densité selon le type d'empreinte si l'on est en présence d'un matériau qui possède plusieurs types d'empreintes différentes.

Selon un mode de réalisation particulier, dans l'étape a), outre l'entité ou molécule empreinte, on peut également ajouter un substrat, de préférence solide. Dans ce mode de réalisation, l'entité ou molécule empreinte, l'amorceur et/ou le polymère à empreintes moléculaires (PEM) au cours de formation peuvent se fixer sur ce substrat.

Au sens de l'invention, par substrat on entend tout matériau solide, soluble ou non, ou tout matériau liquide, pouvant servir de support et permettant la fixation de l'amorceur et/ou le polymère (PEM).

Le substrat peut être avantageusement un substrat de support homogène et isotrope, ou un matériau non-homogène ou de type composite.

Le substrat peut être choisi dans le groupe comprenant le verre, le quartz, le mica, le silicium, le germanium, le carbure de silicium, l'oxyde d'étain-indium, le dioxyde de titane, l'oxyde d'aluminium, un polymère organique éventuellement réticulé notamment le polystyrène, le poly(styrène-co-méthacrylate), le poly(méthylméthacrylate), le poly(acrylonitrile), un polyamide, un polyester, un polyuréthane, le poly(diméthylsiloxane), le polybutadiène, ou un métal notamment l'or, l'argent, le platine, ou un composite de deux ou plusieurs de ces matériaux.

Pour la mise en oeuvre du procédé selon l'invention, tout monomère comprenant au moins une fonction polymérisable par polymérisation radicalaire, peut convenir. L'homme du métier saura aisément choisir les monomères connus en soi qui seront adaptés à la mise en oeuvre du procédé selon l'invention.

Plus particulièrement, dans le procédé selon l'invention, les monomères portent des groupements fonctionnels capables de former des liaisons réversibles avec la ou lesdites entités ou molécules empreintes, par une ou plusieurs liaisons non-covalentes de type hydrogène, ionique van der Waals, ou par interaction hydrophobe, ou par une ou plusieurs liaisons de coordination, ou covalentes.

Les monomères selon l'invention peuvent être des « monomères fixants » éventuellement en mélange avec des « monomères réticulants ».

Au sens de l'invention, par « monomère fixant » on entend un monomère capable de former, de manière réversible, au moins une liaison faible, de coordination et/ou covalente avec l'entité ou molécule empreinte, assurant ainsi une liaison réversible entre ladite entité ou molécule empreinte et le polymère à empreinte moléculaire obtenu à l'issu du procédé.

La liaison faible, de coordination et/ou covalente entre le monomère fixant et l'entité ou molécule empreinte peut se former par une fonction choisie dans le groupe comprenant les fonctions hydroxyle, acide, ester, amide, amine, thiol, éther, amidine, guanidine, aldéhyde et cétone, urée, thiourée, pyridine, imidazole, chélate métallique, ladite fonction étant présente sur le monomère fixant et/ou sur le monomère réticulant.

Par « monomère réticulant », on entend un monomère bi- ou multifonctionnel comprenant au moins deux fonctions polymérisables par polymérisation radicalaire, capable de former de manière permanente, des liaisons covalentes, avec d'autres monomères fixants et/ou réticulants conduisant ainsi à un polymère avec un réseau polymérique tridimensionnel constituant le polymère à empreinte(s) moléculaire(s) (PEM).

Dans l'étape a), les monomères, qu'ils soient fixants ou réticulants, peuvent comporter au moins une fonction vinyle ou une autre fonction éthylénique adaptée à une polymérisation radicalaire.

De préférence, ces monomères comportant au moins une fonction vinyle sont choisis dans le groupe comprenant :
- l'acide méthacrylique, l'acide trifluorométhylacrylique, l'acide acrylique, l'acide itaconique, l'acide styrènesulfonique, l'acide acrylamidopropanesulfonique, l'acide 4-vinylphénylboronique, l'acide 4-vinylbenzoique, le carboxyéthylacrylate, l'hydroxyéthylméthacrylate phosphate, le 2-vinylpyridine, le 4-vinylpyridine, le 1-vinylimidazole, le 4(5)-vinylimidazole, le 2-aminoéthylméthacrylate, le 2-(diméthylamino)éthyl méthacrylate, l'aminoéthylacrylate, le chlorure de 4-vinylbenzyltriméthylammonium, l'hydroxyéthylméthacrylate, le glycidylméthacrylate, l'acrylamide, le méthacrylamide, l'isopropylacrylamide, le 4-vinylbenzamidine, le 4-méthacrylamidobenzamidine, le styrène, le phénylméthacrylate, le cyclohexylméthacrylate, le méthylméthacrylate, le butylméthacrylate, le hexylméthacrylate, le octylméthacrylate, méthylène glycol diméthacrylate, l'hexanediol diméthacrylate, le trisméthylolpropane triméthacrylate, le polyéthylène glycol diméthacrylate, le triéthylène glycoldiméthacrylate, le pentaérythritol triacrylate, le divinylbenzène, l'éthylène bisacrylamide, le méthylène bisacrylamide, le bisacryloyl pipérazine, le N,N-bisacryloylcystamine, le *N*,*N*-1,2-dihydroxyéthylène bisacrylamide, N,O-bisméthacryloyl éthanolamine ;
- ou un mélange de ceux-ci.

Les monomères de l'invention peuvent encore être choisis dans le groupe comprenant des monomères hybrides comprenant une fonction vinyl polymérisable par polymérisation radicalaire et une fonction de type silane polymérisable par un procédé sol-gel, comme par exemple le 3-méthacryloxypropyl-triméthoxysilane.

Par procédé sol-gel, on entend un procédé conduisant à l'élaboration à basse température, de solides amorphes transparents et denses, dans lesquels des espèces moléculaires organiques peuvent être introduites permettant ainsi le développement de nouveaux matériaux hybrides organiques-inorganiques. Ce type de procédé est décrit notamment dans [7].

Comme indiqué précédemment, dans l'étape a), on utilise au moins un amorceur comportant au moins deux fonctions chimiques capables de former des radicaux qui permettent d'amorcer la polymérisation.

Ainsi, l'amorceur utilisé est un amorceur de polymérisation radicalaire comportant avantageusement au moins deux voire plusieurs fonctions chimiques pouvant former des radicaux simultanément. En même temps, cet amorceur peut comporter avantageusement une ou plusieurs fonctions chimiques (F2) lui permettant de se fixer de façon covalente ou non-covalente sur la ou les entités ou molécules empreintes de l'impression moléculaire, ou sur un substrat. De cette façon, la présente invention permet de préparer des polymères à empreinte(s) moléculaire(s) de façon dense et localisée sur ou autour de la ou des entités ou molécules empreintes.

Comme indiqué précédemment, dans l'étape a), on utilise au moins un amorceur comportant au moins deux fonctions chimiques (F1) capables de former des radicaux qui permettent d'amorcer la polymérisation des monomères sur ou autour de l'entité ou molécule empreinte.

L'amorceur peut comporter jusqu'à 64 fonctions (F1), de préférence de 2 à 16, et plus préférentiellement de 4 à 8 fonctions chimiques (F1) capables de former des radicaux qui permettent d'amorcer la polymérisation des monomères sur ou autour de l'entité ou molécule empreinte.

Les fonctions chimiques (F1) de l'amorceur capable de former des radicaux permettant d'amorcer la polymérisation peuvent être apportées par exemple, par un composé choisis dans le groupe comprenant :
- les éthers de benzoïne notamment le méthyléther de benzoïne, féthyléther de benzoïne, le butyléther de benzoïne, l'isopropyléther de benzoïne, les acétophénones notamment la 2,2-diméthoxy-2-phényl acétophénone, le 2-hydroxy-2-méthyl-1-phényl-propan-1-one ou le 2-méthyl-1-[4-(méthylthio)phényl]-2-morpholinopropan-1-one, les benzophénones substitués ;
- les peroxydes substitués notamment le bis(tert-butyl cyclohexyl)peroxydicarbonate, les dérivés des phosphine-oxydes notamment le bis(2,4,6-triméthylbenzoyl)-phénylphosphine-oxyde, les amino-cétones, les oxysulfonyl-cétones, les sulfonyl-cétones ou l'un de ces dérivés, et les composés de type azo notamment l'azo-bis-isobutyronitrile, l'azo-bis-dimethylvaléronitrile, l'acide 4,4'-azobis-cyanovalérique, le 2,2'-azobis(2-amidinopropane) ;
- les hydroxy-alkylamines notamment la méthyldiéthanolamine, les benzylamines, les dérivés d'aniline, le paradiméthylaminobenzoate d'éthyle, la N-phénylglycine (NPG) ;
- l'acide ascorbique ;
- un dérivé de dithiocarbamate notamment le benzyl-N,N-diméthyldithio-carbamate, le benzyl-N,N-diéthyldithiocarbamate, le benzyl-N,N-di(carboxyméthyl)dithiocarbamate, le benzyl-N,N-di(2-hydroxyéthyl)dithio-carbamate, le benzyl-N-méthyl-N-(2-hydroxyéthyl)dithiocarbamate, le benzyl-N-méthyl-N-carboxyméthyldithiocarbamate, le di(benzylester) du benzyl-N,N-di(carboxyméthyl)dithiocarbamate.

Cette liste n'étant pas limitative, les fonctions chimiques (F1) de l'amorceur peuvent également être apportées par tout autre composé capable de former des radicaux permettant d'amorcer la polymérisation.

L'amorceur peut également comporter au moins une seconde fonction chimique (F2) permettant d'établir une liaison faible, de coordination et/ou covalente entre ledit amorceur et l'entité ou molécule empreinte.

Cette fonction (F2) peut assurer ainsi une liaison réversible ou irréversible entre le polymère à empreinte(s) moléculaire(s) et l'entité ou molécule empreinte.

Lorsque l'on ajoute un substrat, en particulier un substrat solide soluble ou non, ou un substrat liquide dans l'étape a), cette seconde fonction (F2) peut également établir une liaison faible, de coordination et/ou covalente entre l'amorceur et ledit substrat.

Cette seconde fonction chimique (F2) peut être choisie dans le groupe comprenant l'hydroxyle, l'acide carboxylique, l'ester carboxylique, le thioester, l'amide, l'amine, le thiol, l'éther, l'amidine, la guanidine, l'urée, la thiourée, l'aldéhyde, la cétone, l'alkyle, le phényle, le benzyle, l'acide iminodiacétique, l'acide nitrilotriacétique, l'acide éthylène diaminotétraacétique, un chélate métallique notamment l'acide iminodiacétiquè-Cu, l'acide iminodiacétique-Co, l'acide iminodiacétique-Ni, l'acide iminodiacétique-Zn, l'acide nitrilotriacétique-Ni.

De préférence, les fonctions chimiques de l'amorceur sont l'acide carboxylique, la benzamidine.

Selon une première variante, ledit procédé met en oeuvre la fixation de façon suffisamment dense de l'amorceur de polymérisation sur un substrat. On obtient ainsi un film uniforme et homogène en épaisseur, l'épaisseur étant variable en fonction du temps de polymérisation.

Selon une seconde variante, ledit procédé met en oeuvre la fixation de façon assez peu dense de l'amorceur de polymérisation sur un substrat. On obtient ainsi des nanostructures ou microstructures attachés au substrat, de dimensions uniformes et de distribution homogène. De cette manière on obtient un produit dit « structuré », ce qui permet de répondre aux demandes variées intéressant les différentes branches de l'industrie qui utilisent ce type de produit. Ceci a comme avantage de permettre la fabrication de structures optimisant la transduction d'un capteur tout en augmentant la surface spécifique du PEM.

Selon une troisième variante, ledit procédé met en oeuvre l'utilisation de l'amorceur de polymérisation en solution avec les monomères et l'entité ou la molécule empreinte. On obtient ainsi des polymères à empreinte(s) moléculaire(s) sous forme de particules sphériques ou non-sphériques de taille comprise entre 10 nm et 100 µm, ou des nanogels solubles de taille inférieure à 1 µm.

Dans une quatrième variante de l'invention, ledit procédé met en oeuvre de façon avantageuse l'utilisation d'un amorceur portant une ou plusieurs fonctions chimiques supplémentaires lui permettant de former des liaisons covalentes ou non-covalentes avec l'entité ou la molécule empreinte. De cette façon, l'amorceur de polymérisation joue en même temps le rôle d'un monomère fixant, et les chaînes de polymère se forment à partir de l'entité ou de la molécule empreinte et ceci de façon localisée. L'entité ou molécule empreinte est ainsi enrobée complètement ou partiellement par un polymère (PEM) suffisamment dense. Selon cette variante l'amorceur de polymérisation est en solution avec les monomères et l'entité ou la molécule empreinte.

Dans l'étape a), l'amorceur peut être en mélange avec un composé sensibilisateur choisi dans le groupe comprenant :
- les acridines notamment l'Acriflavine^{®} ou l'Acridine Orange^{®}, les phénazines notamment la Safranine O^{®}, les oxazines, les thiazines notamment le Bleu de Méthylène ou la thionine, les xanthènes notamment l'Eosine Y^{®}, le Rose Bengale^{®} ou l'Erythrosine^{®}, les rhodamines, les thioxanthènes, les polyméthines ;
- les dérivés de la thioxanthone notamment l'isopropylthioxanthone ou le chlorothioxanthone, les coumarines ou leurs dérivés notamment les cétocoumarines.

Avantageusement, la mise en contact de l'étape a) se fait en présence d'au moins un agent porogène apte à former des pores dans le polymère à empreinte(s) moléculaire(s) (PEM) que l'on souhaite obtenir, de préférence par adjonction d'un solvant ou d'un mélange de solvant(s) porogène(s), ou encore par adjonction d'un agent porogène dans un solvant dans une solution où sont présentes des substances réactives.

On obtient ainsi un polymère à empreinte(s) moléculaire(s) de structure poreuse, ce qui facilite l'accès aux et/ou le départ des entités ou molécules empreintes du polymère (PEM).

De préférence, le procédé selon l'invention met en oeuvre au moins un agent porogène conférant au polymère (PEM) une structure poreuse dont le diamètre des pores est compris entre 0,1 nm et 10 µm, et de préférence entre 1 nm et 1 µm. Plus préférentiellement, le solvant porogène peut être un solvant aqueux ou non-aqueux, organique ou non. De préférence la mise en contact de l'étape a) se fait en présence d'au moins un solvant porogène choisi dans le groupe comprenant :
- le benzène, le toluène, le xylène, les solvants chlorés notamment le chloroforme, le dichlorométhane, le dichloroéthane et le 1,1,2,2,-tétrachloroéthane, et/ou les solvants perfluorés, notamment le hexafluorocyclohexane, les éthers notamment le tétrahydrofurane, le diéthylène glycol diméthyle éther et le triéthylène glycol diméthyle éther, les amides notamment le diméthylformamide et le formamide, les nitriles notamment l'acétonitrile et le caprylonitrile, les cétones notamment l'acétone, les esters notamment l'acétate d'éthyle, les alcanes notamment l'hexane et le heptane, les alcools notamment le méthanol, l'éthanol, l'iso-propanol, le butanol, le décanol, l'eau ;
- ou un mélange de ceux-ci.

Le ou solvants précités peuvent, en outre, comprendre un agent co-porogène en particulier de nature polymérique ou autre, de préférence un polymère linéaire soluble dans ledit solvant, notamment le polyéthylène glycol, le polyvinylalcool, le polyvinylacétate, le polystyrène, le polyméthylméthacrylate. Cet agent aura pour fonction d'induire et d'accélérer la formation des pores. De tels agents sont également décrits dans le document [8].

Comme indiqué précédemment, dans l'étape b) on effectue la polymérisation sous l'action d'un rayonnement lumineux de longueur d'onde visible ou UV ou sous l'action d'un apport d'énergie thermique pour former un polymère à empreinte(s) moléculaire(s) (PEM) sur ou autour de ladite entité ou molécule empreinte.

Selon une autre caractéristique, dans l'étape b) le procédé selon l'invention met en oeuvre des radiations ou rayons lumineux sous la forme d'un rayon ou faisceau de rayons lumineux de longueur d'onde visible ou UV.

Ainsi, dans l'étape b) on effectue la polymérisation sous l'action d'un rayonnement lumineux de longueur d'onde visible ou UV d'une longueur d'onde comprise entre 100 nm et 1200 nm. De préférence, la longueur d'onde est comprise entre 150 nm et 750 nm, et plus préférentiellement entre 250 nm et 550 nm.

Selon une autre caractéristique, dans l'étape b) la polymérisation est mise en oeuvre par un apport d'énergie thermique qui permet l'activation de l'amorceur de polymérisation. Ceci peut se faire par exemple en plaçant le mélange de polymérisation dans une enceinte thermostatée dont la température peut être réglée.

Ainsi, on peut effectuer la polymérisation dans l'étape b) sous un apport d'énergie thermique à une température comprise entre 0°C et 200°C. De préférence, la température est comprise entre 25°c et 100°C, et plus préférentiellement entre 40°C et 70°C.

De préférence la polymérisation se fait sans modifier chimiquement la ou les entités ou molécules empreintes.

Comme indiqué précédemment, dans l'étape c) du procédé selon l'invention, on élimine le ou les monomères non polymérisés et/ou tout ou partie de l'entité ou molécule empreinte du polymère formé en b) avec un solvant ou un mélange de solvant(s) approprié(s).

Le solvant peut être choisi dans le groupe comprenant l'eau, le méthanol, l'éthanol, l'iso-propanol, l'acétonitrile, le chloroforme, l'heptane, le toluène, l'acide acétique, ou leur mélange, ou le CO₂ supercritique. De préférence, le solvant est un mélange de méthanol et d'acide acétique.

Outre les solvants indiqués, l'entité ou molécule empreinte peut également être éliminé du polymère formé en b) avec un autre agent ou un autre traitement susceptible de faciliter l'élimination de l'entité ou molécule empreinte.

Selon une variante de l'invention, Dans l'étape c), outre le solvant ou le mélange de solvants, on peut éliminer l'entité ou molécule empreinte du polymère formé en b) avec un solvant ou un mélange de solvants contenant un autre agent choisi dans le groupe comprenant :
- un détergent notamment le dodécylsulfate de sodium, le bromure de cétyl triméthylammonium, le Triton X-100^{®}, l'Empigen BB^{®};
- l'urée ;
- l'acide trifluoroacétique ;
- la triéthylamine ;
- une enzyme hydrolytique choisie dans le groupe comprenant une peptidase ou une protéase, notamment la trypsine ou la protéinase K ;
- une nucléase, notamment la nucléase de *Staphylococcus aureus* ou la ribonucléase A ;
- une lipase, notamment la lipase du pancréas de porc ou la lipase XIII de *Pseudomonas sp. ;*
- une glycosidase, notamment la beta-glucosidase, la alpha-glucosidase, ou la beta-galactosidase ;
- un sel notamment le thiosulfate de sodium.

L'homme du métier reconnaît le ou les solvants approprié(s) et, le cas échéant, l'agent, notamment parmi ceux cités précédemment, pour réaliser l'étape c) dans laquelle on élimine le ou les monomères non polymérisés et/ou tout ou partie de l'entité ou molécule empreinte du polymère formé en b).

Selon une autre variante de l'invention, outre le solvant, on élimine l'entité ou molécule empreinte du polymère formé en b) en appliquant un champs électrique compris de préférence entre 1 V/cm et 100 V/cm. Dans cette variante, le polymère (PEM) est retenu par une membrane sémiperméable que l'entité ou molécule empreinte peut traverser.

L'invention a également pour objet un polymère à empreinte(s) moléculaire(s) (PEM) susceptible d'être obtenu par le procédé de l'invention.

Le polymère à empreinte(s) moléculaire(s) susceptible d'être obtenu par la mise en oeuvre du procédé selon l'invention peut se présenter sous la forme d'un film d'une épaisseur comprise entre 1 nm et 100 µm, de préférence sous la forme d'un film d'épaisseur constante ou sensiblement constante.

Il peut se présenter également sous la forme d'un film spatialement structurée, de préférence présentant des motifs géométriques dont les diamètres ou dimensions latérales sont comprises entre 1 nm et 10 µm.

Ledit polymère peut aussi se présenter sous la forme de particules sphériques notamment sous la forme de microbilles ou nanobilles sphériques en suspension, ou de microgels ou nanogels en solution, dont le diamètre est compris de préférence entre 1 nm et 100 µm.

Le polymère à empreinte(s) moléculaire(s) (PEM) susceptibles d'être obtenu selon le procédé de l'invention, peut avoir une structure poreuse dont le diamètre des pores peut être comprise entre 0,1 nm et 10 µm.

Un autre objet de l'invention est l'utilisation d'un polymère susceptible d'être obtenu par le procédé de l'invention dans la réalisation des biocapteurs biomimétiques, des biopuces biomimétiques, des capteurs chimiques, des dispositifs de séparation par adsorption spécifique, en tant que revêtement pour le relargage notamment de produits actifs.

Plus particulièrement, la présente invention concerne l'utilisation d'un polymère à empreinte(s) moléculaire(s) obtenu par la mise en oeuvre du procédé selon l'invention pour la fabrication d'un détecteur qualitatif et/ou quantitatif de la présence d'au moins une entité ou molécule cible dans un échantillon à analyser-ainsi que le détecteur obtenu par la mise en oeuvre du procédé selon l'invention.

La présente invention concerne aussi un polymère à empreinte(s) moléculaire(s) obtenu par la mise en oeuvre du procédé selon l'invention pour la fabrication d'un capteur réversible d'au moins une entité ou molécule cible ainsi que le capteur réversible obtenu par la mise en oeuvre du procédé selon l'invention.

Enfin, la présente invention concerne l'utilisation d'un polymère à empreinte(s) moléculaire(s) obtenu par la mise en oeuvre du procédé selon l'invention pour la fabrication d'un dispositif de séparation par affinité pour au moins une entité ou molécule cible ainsi que le dispositif de séparation par affinité obtenu par la mise en oeuvre du procédé selon l'invention.

La présente invention permet de résoudre le problème de la mise en forme, ainsi que le problème de morphologie et de structure interne, de matériaux ou polymères à empreintes moléculaires (PEM) avec comme objectif de fabriquer notamment des capteurs chimiques au sens précité dont le fonctionnement repose sur le principe de la reconnaissance moléculaire.

Le procédé de préparation de PEM permet également d'obtenir une structuration multi-échelle, comme une microstructuration et une nanostructuration simultanées. La méthode permet également d'obtenir une multiple nanostructuration ou une multiple microstructuration, comme une superposition de plusieurs films de PEM sur une surface, ou des structures sphériques ou hémi-sphériques de type coeur-coquille (core-shell en anglais) ou coeur-multicoquille (core-multishell en anglais).

Ceci permet d'optimiser à la fois la sélectivité de la reconnaissance et la sensibilité de la réponse d'un dispositif basé sur un tel matériau PEM nanostructuré à deux échelles. Ceci permet également d'optimiser les propriétés du matériau, ou d'incorporer des entités servant à son identification spécifique par voie physico-chimique.

L'invention concerne également les amorceurs de formules *D1* (figure 2), *D2* et *D6* (figure 4), *D3* (figure 9), *D4* (figure 13), *D5* (figure 14) ainsi que leur utilisation pour la préparation d'un polymère à empreinte(s) moléculaire(s) par polymérisation radicalaire.

D'autres avantages pourront encore apparaître à l'homme du métier à la lecture des exemples ci-dessous, illustrés par les figures annexées, donnés à titre illustratif.

### Brève description des figures

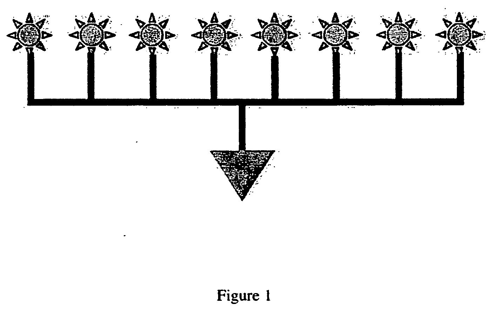

- La figure 1 représente un schéma simplifié de la structure générale des amorceurs de polymérisation photo ou thermosensibles de la présente invention.

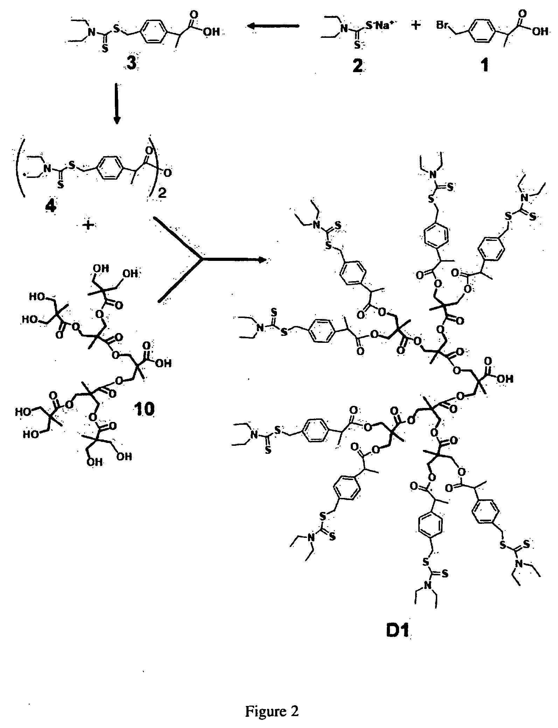

- La figure 2 représente la structure chimique et la voie de synthèse de la molécule *D1*, la molécule amorceur spécifique décrite dans l'exemple 1. *D1* est synthétisée sur la base d'une structure dendritique dans laquelle la fonction (F2) au coeur permettant la formation de liaisons covalentes ou non-covalentes est une fonction carboxyle, et dans laquelle les fonctions (F1) à la périphérie permettent de générer des radicaux réactifs et donc d'amorcer une polymérisation radicalaire sont 8 fonctions benzyldiéthyldithiocarbamate (amorceur de type iniferter pour polymérisation radicalaire vivante).

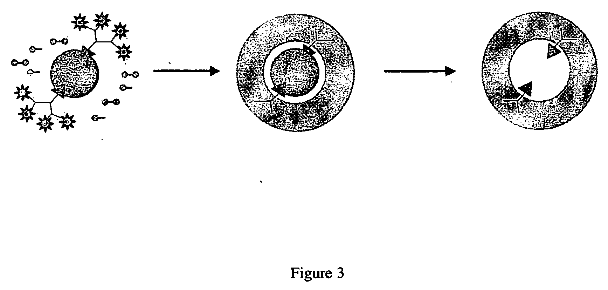

- La figure 3 représente un schéma simplifié de la préparation des polymères (PEM) en solution ou en suspension selon l'invention (exemple 2).

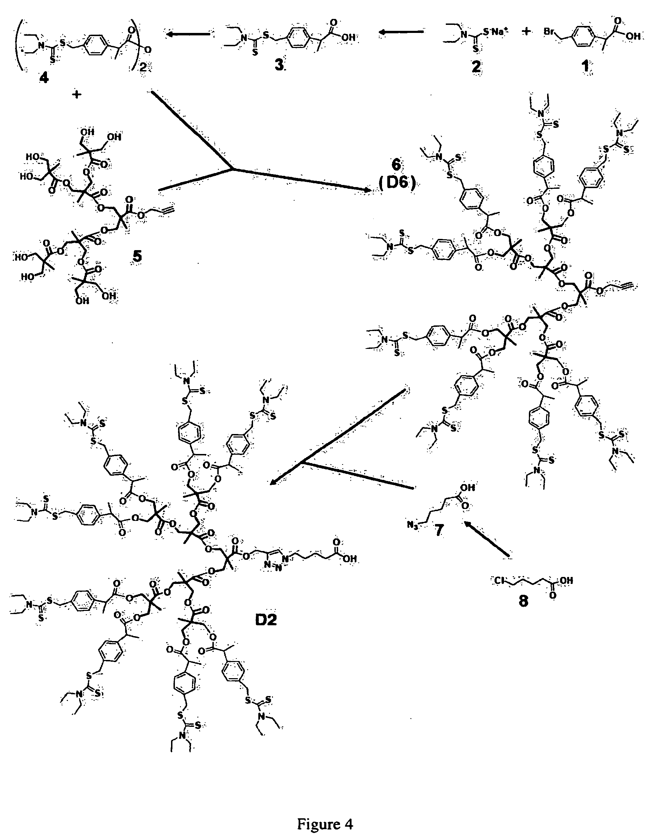

- La figure 4 représente la structure chimique et la voie de synthèse de la molécule *D2*, la molécule amorceur spécifique utilisé dans l'exemple 2, ainsi que celle de la molécule *D6* (aussi identifiée comme 6). *D2* est synthétisée sur la base d'une structure dendritique dans laquelle la fonction (F2) au coeur permettant la formation d'une liaison covalente ou non-covalente rattachée au dendron (génération 3) est une fonction carboxyle, et dans laquelle les fonctions (F1) à la périphérie permettent de générer des radicaux réactifs et donc d'amorcer une polymérisation radicalaire sont 8 fonctions benzyldiéthyldithiocarbamate (amorceur de type iniferter pour polymérisation radicalaire vivante). La molécule 6 ou *D6* est synthétisée sur la base d'une structure dendritique dans laquelle la fonction (F2) au coeur est une fonction acétylène permettant une fixation par « chimie clic » (« click chemistry » en anglais) sur une autre entité comportant un groupement azide, et dans laquelle les 8 fonctions (F1) à la périphérie sont des fonctions benzyldiéthyldithiocarbamate permettant de générer des radicaux réactifs, et donc d'amorcer une polymérisation radicalaire.

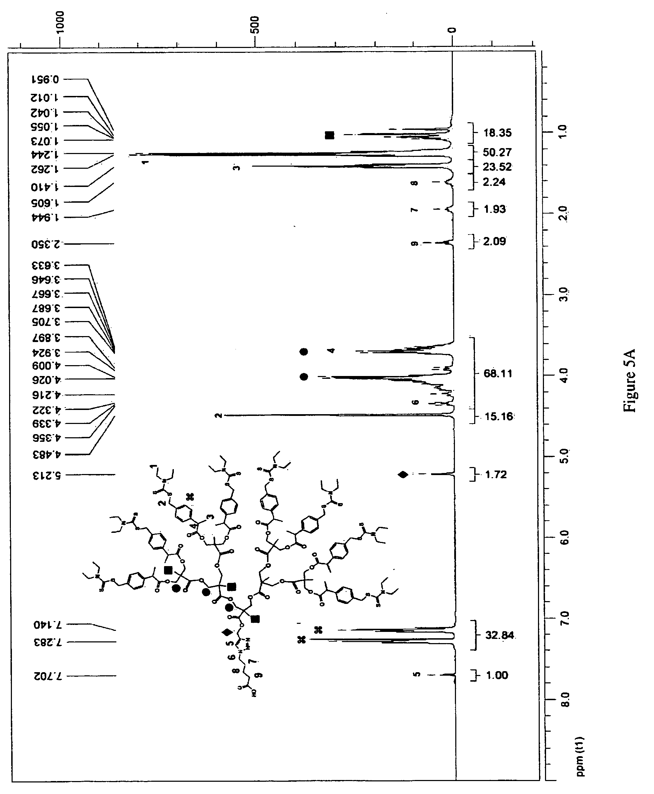

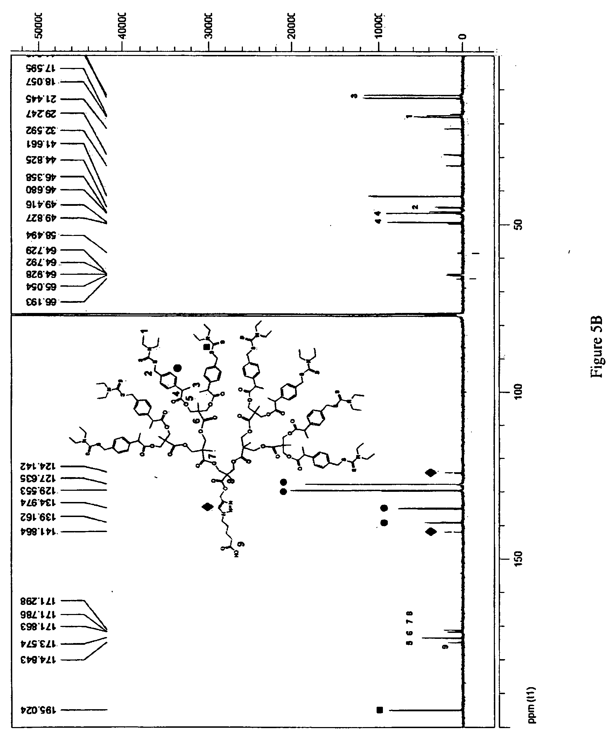

- Les figures 5A et 5B montrent les spectres de résonance magnétique nucléaire de proton (figure 5A) et de carbone (figure 5B) de *D2.*

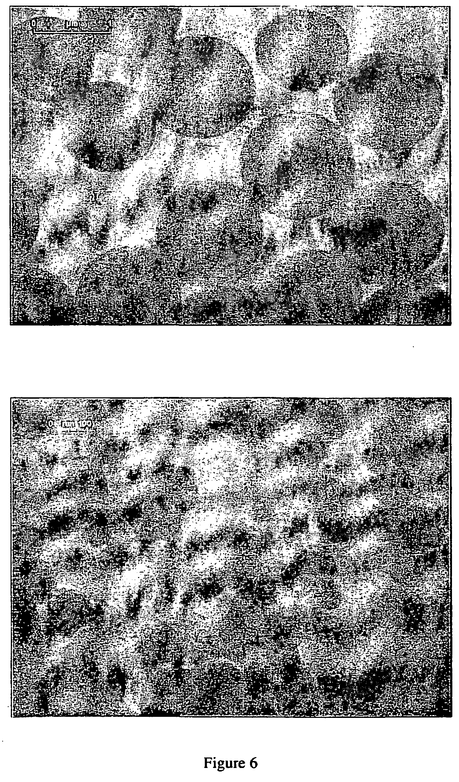

- La figure 6 représente un cliché pris, par imagerie en microscopie électronique en transmission, de particules de PEM obtenu par le procédé selon l'invention et selon le schéma de la figure 3 (en haut), selon l'exemple 2 ; et pour comparaison, un cliché pris, par imagerie en microscopie électronique en transmission de particules de PEM obtenu en utilisant un amorceur de polymérisation qui contient une seule fonction permettant de générer des radicaux, le benzyldiméthyldithiocarbamate (en bas), le nombre total de fonctions dithiocarbamate étant le même dans les deux cas.

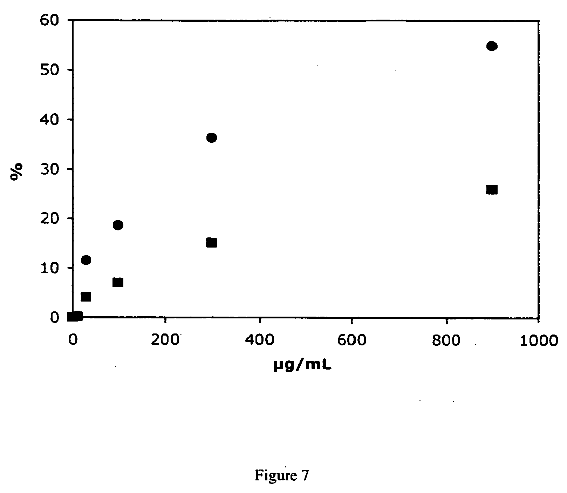

- La figure 7 représente un graphe montrant la variation de l'adsorption de la molécule empreinte radiomarquée ³H-S-propranolol sur un polymère à empreintes moléculaires (PEM) (symboles ronds) ou sur un polymère contrôle non-imprimée correspondant (symboles carrés), en fonction de la variation de la concentration des polymères, selon l'exemple 2.

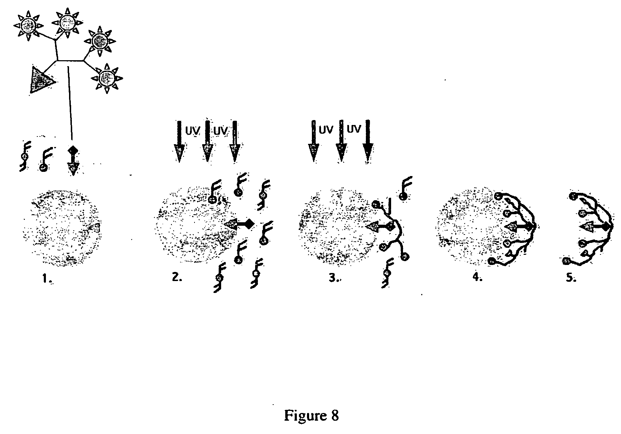

- La figure 8 représente un schéma simplifié d'une variante du procédé visant à fabriquer un polymère à empreintes moléculaires (PEM) sous forme de nanoparticules dont le diamètre, compris entre 20 nm et 500 nm, est fonction du temps de polymérisation pour fixer spécifiquement la trypsine, selon l'exemple 3.

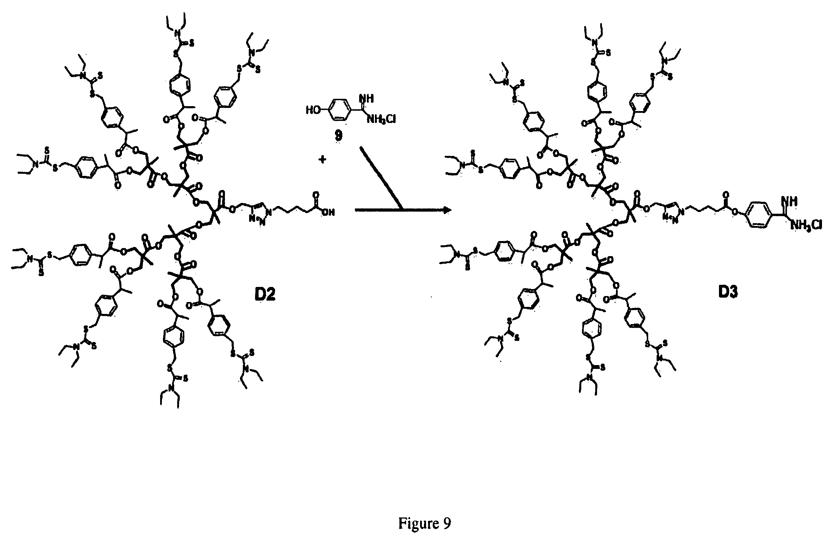

- La figure 9 représente la structure chimique et la voie de synthèse de la molécule *D3*, molécule amorceur spécifique utilisé dans l'exemple 3. *D3* est synthétisée sur la base d'une structure dendritique (dendron génération 3) dans laquelle la fonction (F2) au coeur permettant un couplage par liaisons covalentes ou non-covalentes est une fonction benzamidine rattachée au dendron via un bras espaceur, et dans laquelle les fonctions (F1) à la périphérie permettant de générer des radicaux réactifs, et donc d'amorcer une polymérisation radicalaire sont 8 fonctions benzyldiéthyldithiocarbamate (amorceur de type iniferter pour polymérisation radicalaire vivante).

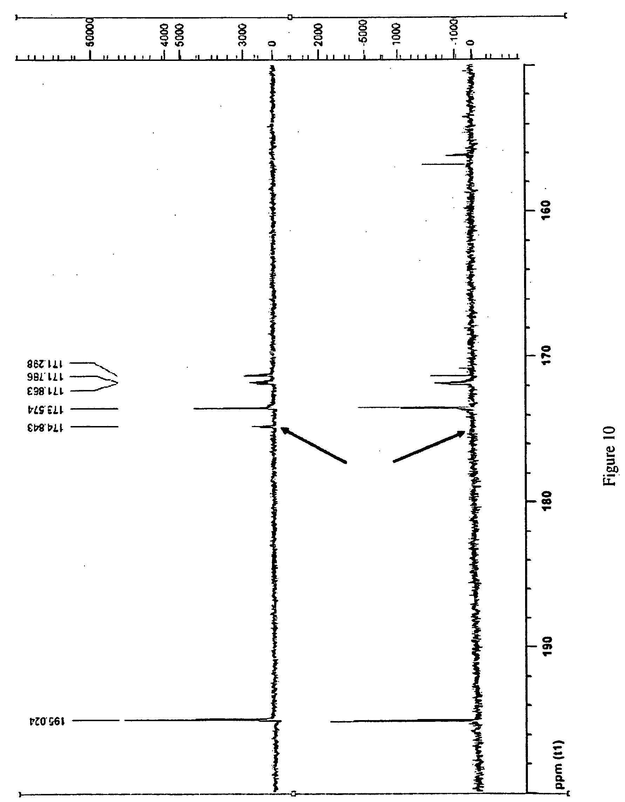

- La figure 10 montre le spectre de résonance magnétique nucléaire de carbone de *D3* (en bas) et, pour comparaison, le spectre de résonance magnétique nucléaire de carbone de *D2* avant couplage avec le composé 9 (en haut). Les flèches indiquent la disparition du signal de la fonction -COOH libre suite au couplage de *D2* avec le composé 9.

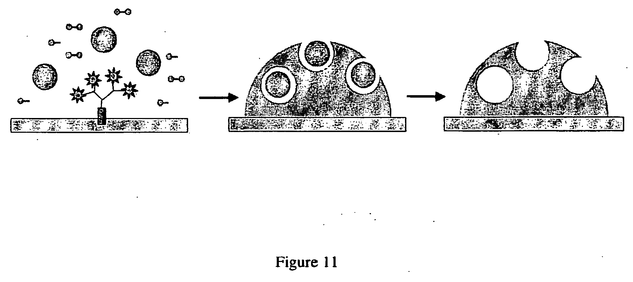

- La figure 11 représente un schéma simplifié d'une variante du procédé utilisé à la figure 3, où l'amorceur est immobilisé sur une surface (exemple 4).

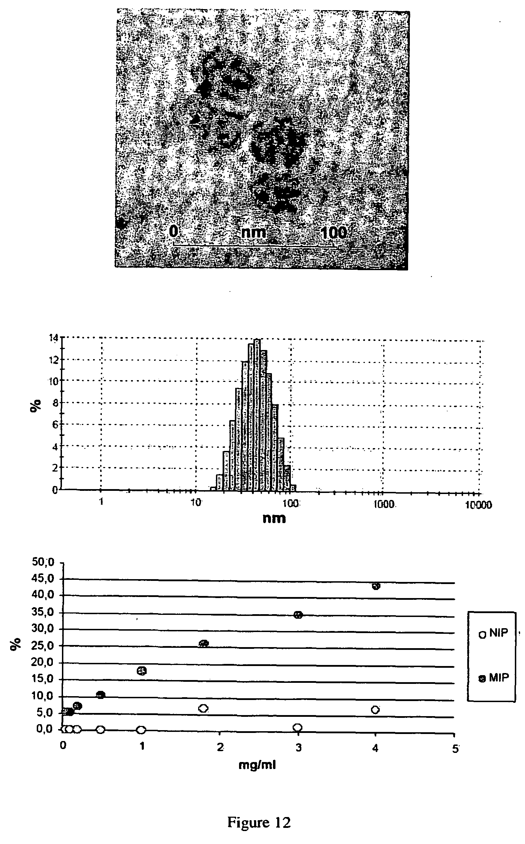

- La figure 12 représente un cliché pris, par imagerie en microscopie électronique en transmission, de particules de PEM obtenues par le procédé selon l'invention et selon l'exemple 5 (en haut) ; le graphe du milieu montre le résultat d'une analyse des particules par diffusion dynamique de lumière : la variation du pourcentage de l'intensité en fonction de la taille de particule ; le graphe du bas montre la variation de l'adsorption de la molécule empreinte radiomarquée ³H-S-propranolol sur un polymère à empreintes moléculaires (PEM) spécifique pour le S-propranolol (symboles ronds remplis) ou sur un polymère contrôle non-imprimé correspondant (symboles ronds vides), en fonction de la variation de la concentration des polymères, selon l'exemple 5.

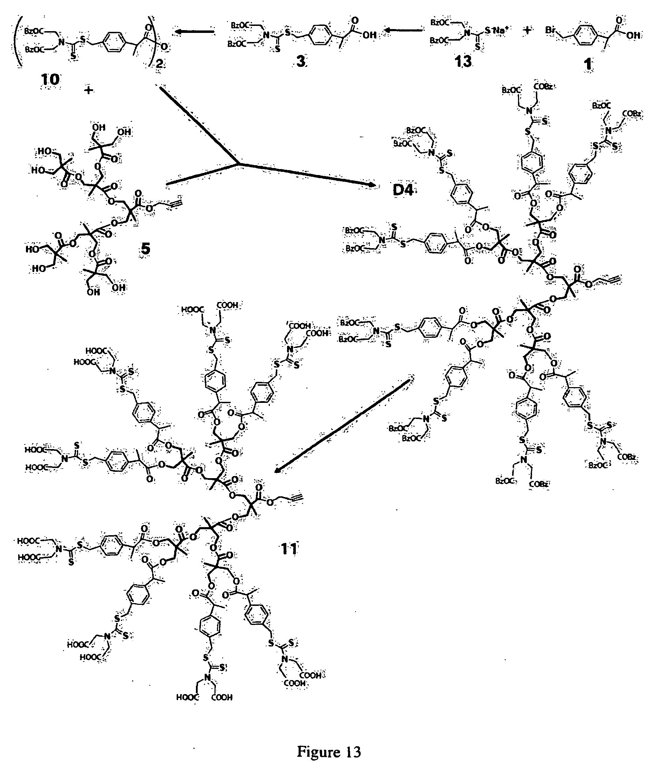

- La figure 13 représente la structure chimique et la voie de synthèse de la molécule *D4*, la molécule amorceur spécifique décrite dans l'exemple 1. *D4* est synthétisée sur la base d'une structure dendritique dans laquelle les fonctions (F1) à la périphérie permettent de générer des radicaux réactifs et donc d'amorcer une polymérisation radicalaire sont 8 fonctions di(benzylester) du benzyl-N,N-di(carboxyméthyl)dithiocarbamate (amorceur de type iniferter pour polymérisation radicalaire vivante). A partir de la molécule *D4*, la molécule 11 peut être obtenue par hydrogénolyse catalytique des fonctions ester benzylique.

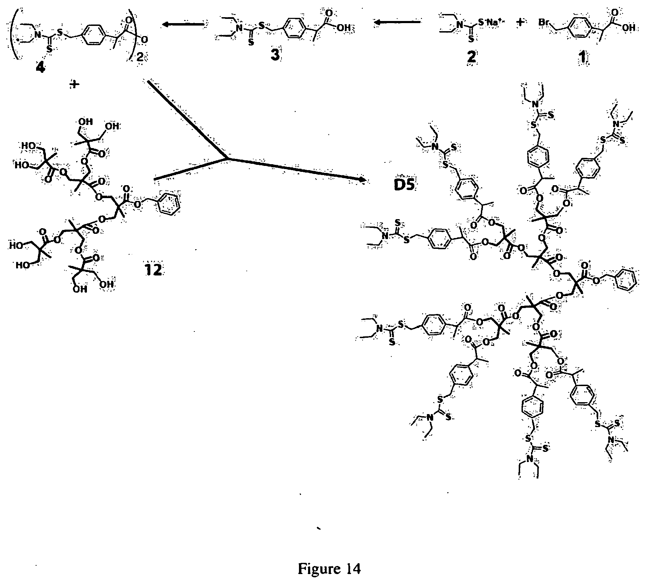

- La figure 14 représente la structure chimique et la voie de synthèse de la molécule *D5*, la molécule amorceur spécifique décrite dans l'exemple 1. *D5* est synthétisée sur la base d'une structure dendritique dans laquelle les fonctions (F1) à la périphérie permettent de générer des radicaux réactifs et donc d'amorcer une polymérisation radicalaire sont 8 fonctions benzyldiéthyldithiocarbamate (amorceur de type iniferter pour polymérisation radicalaire vivante).

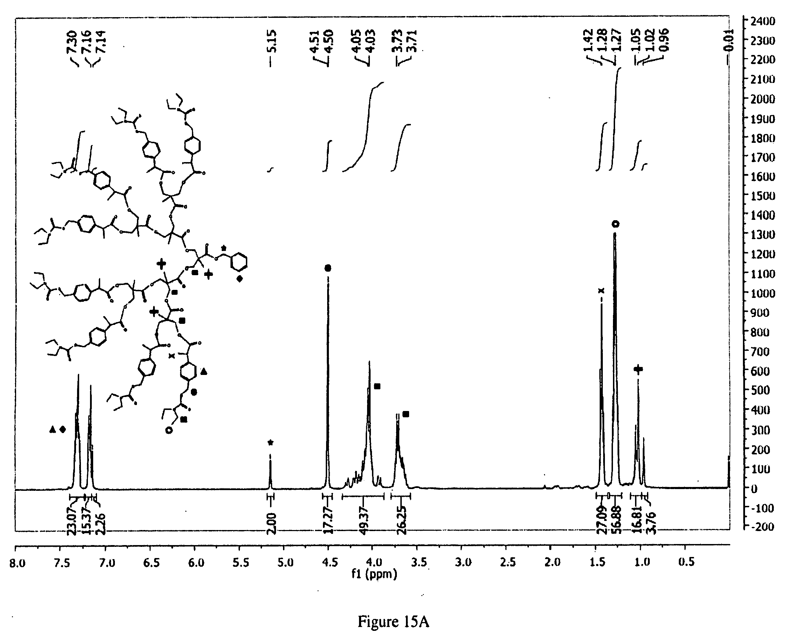

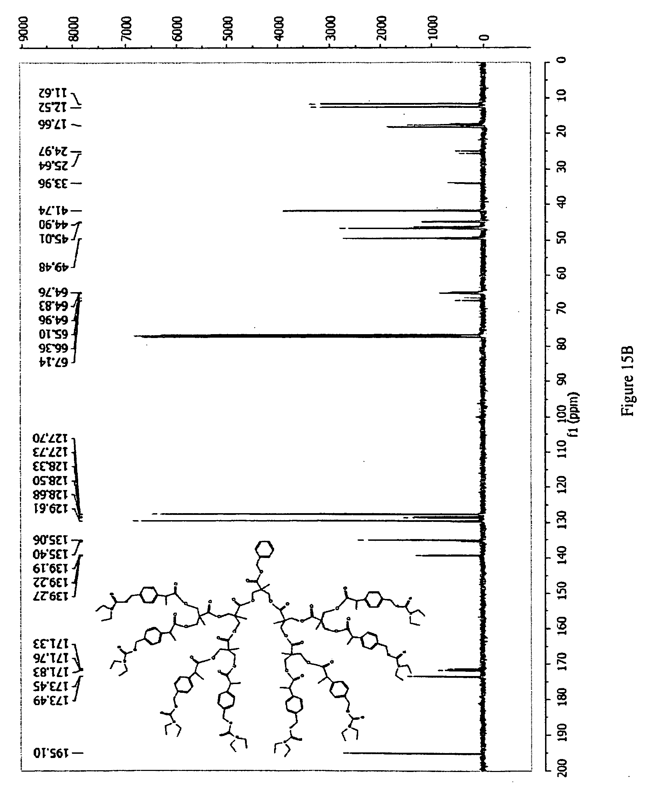

- Les figures 15A et 15B montrent les spectres de résonance magnétique nucléaire de proton (figure 15A) et de carbone (figure 15B) de *D5.*

### EXEMPLES

Dans les exemples, l'une des molécules empreintes utilisées comme modèle pour démontrer l'impression du matériau polymère est le S-propranolol (beta-bloquant). L'intérêt de cette molécule réside entre autres dans sa chiralité : si l'un des énantiomères est utilisé pour générer l'empreinte, l'autre constitue un excellent contrôle de la sélectivité du polymère (PEM). Une autre molécule empreinte utilisée est la trypsine. L'intérêt de la trypsine est qu'il s'agit d'une protéine qui est une molécule nettement plus grande que le S-propranolol.

La sélectivité des polymères bruts synthétisés pour une molécule donnée peut être caractérisée à l'aide de différents moyens : utilisation de marqueurs fluorescents ou radioactifs (tests à l'équilibre), chromatographie en phase liquide, ou par des méthodes spectroscopiques basées sur la fluorescence, l'absorption de lumière ultraviolette, visible ou infrarouge, ou sur l'effet Raman.

Il a été validé que le procédé selon l'invention peut être étendu à d'autres mélanges polymérisables compatibles avec la technique d'impression moléculaire. L'épaisseur d'un film et, le cas échéant, la dimension latérale des micro ou nanostructures de polymères, et le diamètre des micro ou nanobilles sphériques de PEM fabriqués sont directement reliés au temps d'irradiation et à la puissance du faisceau utilisé, ou au temps de chauffage pour l'amorçage par apport d'énergie thermique. Dans ces conditions, ces dimensions peuvent être facilement fixées entre quelques nm et quelques µm.

### Exemple 1 : Multiamorceurs de polymérisation selon l'invention

La figure 1 montre une représentation schématique du multiamorceur de polymérisation qui porte également une fonction lui permettant de se lier à un substrat ou à l'entité ou molécule empreinte.

### 1.1. Amorceur D1 :

Un exemple de réalisation d'un multiamorceur est la molécule *D1* décrite en figure 2. Cette molécule est basée sur une structure dendritique (dendron de génération 3), contenant dans le coeur une fonction (F2) qui est une fonction COOH permettant de coupler ou fixer, par des liaisons covalentes ou non-covalentes (liaisons hydrogènes ou ioniques), la molécule sur un substrat, une surface, l'entité ou molécule empreinte, et contenant sur sa périphérie des fonctions (F1) amorceurs de polymérisation radicalaire vivante de type iniferter (benzyldiéthyldithiocarbamate).

### Synthèse de la molécule D1 :

Les étapes de synthèse, les réactifs utilisés et la structure de la molécule *D1* obtenue sont illustrés dans la figure 2.

La molécule *1* (1 équivalent, 1 mmol, Sigma-Aldrich 530360) et la molécule 2 (1,2 équivalents, 1,2 mmol, Sigma-Aldrich 228680) sont dissous dans 20 mL de tétrahydrofurane (THF) sous atmosphère d'azote. La réaction évolue pendant 4 heures sous agitation à 70°C sous atmosphère d'azote. La phase organique est lavée par une solution saturée de chlorure de sodium, séchée sur sulfate de magnésium et concentrée. Une chromatographie sur gel de silice du résidu permet d'isoler le produit attendu 3. La molécule 3 (0,9 mmol) est mise en réaction avec le N,N-dicyclohexylcarbodiimide (1,5 équivalent, 1,8 mmol, Sigma-Aldrich 36650) dans le dichlorométhane (15 mL) à 25°C pendant 2 heures. Un précipité se forme et est éliminé par filtration sur fritté. Le filtrat est ensuite concentré à l'évaporateur rotatif pour donner l'anhydride 4. En présence de 4-(diméthylamino)pyridine (0,2 équivalents/-OH, 1,3 mmol) et de pyridine (5 équivalents/-OH, 32 mmol), la molécule 4 (9,6 équivalents, 7,7 mmol, 1,5 équivalents/-OH) et le dendron 10 (1 équivalent, 0,8 mmol, Polymer Factory PFd-G3-COOH-OH) sont dissous dans le dichlorométhane (20 mL). La réaction évolue à 25°C pendant 24 heures. La phase organique est rincée avec une solution aqueuse de carbonate de sodium 10% et une solution saturée de chlorure de sodium, séchée sur sulfate de magnésium, filtrée puis concentrée sous pression réduite pour donner une huile. Cette dernière est purifiée par chromatographie sur gel de silice (heptane/acétate d'éthyle 40/60) pour isoler le dendron *D1* (0,4 mmol) avec un rendement global de 40%.

### 1.2. Amorceurs D2 et D6:

Un autre exemple de réalisation d'un multiamorceur est la molécule D2 dont la structure est décrite dans la figure 4. Cette molécule est basée sur une structure dendritique (dendron de génération 3), comprenant :
   - au coeur une fonction (F2) qui est une fonction acide carboxylique permettant une fixation par liaison covalente ou non-covalentes et rattachée au dendron via un bras, et
   - à la périphérie 8 fonctions (F1) qui sont des fonctions benzyldiéthyldithiocarbamate permettant de générer des radicaux réactifs, et donc d'amorcer une polymérisation radicalaire (amorceur de type iniferter pour polymérisation radicalaire vivante).

La molécule *D6* (aussi identifiée comme 6) dont la structure est décrite dans la figure 4, est un autre exemple d'un multiamorceur. Cette molécule est basée sur une structure dendritique (dendron de génération 3), comprenant au coeur une fonction (F2) qui est une fonction acétylène permettant une fixation par « chimie clic » (« click chemistry » en anglais) sur une autre entité comportant un groupement azide. Tout comme dans D2, dans la molécule 6 ou *D6*, les 8 fonctions (F1) qui sont à la périphérie, sont des fonctions benzyldiéthyldithiocarbamate permettant de générer des radicaux réactifs, et donc d'amorcer une polymérisation radicalaire.

La chimie clic permet de développer un ensemble de réactions puissantes, reproductibles, sélectives et modulables. Les principales réactions click consistent à former des liaisons carbone-hétéroatome énergétiquement très favorables [11,12].

Les étapes de synthèse, les réactifs utilisés et la structure des molécules *D2* et 6 ou *D6* obtenues sont illustrés en figure 4.

La préparation des molécules 3 et 4 est réalisée selon le protocole décrit pour l'amorceur *D1*. A une solution de la molécule 4 (9,6 équivalents, 7,7 mmol, 1,5 équivalents/-OH) dans le dichlorométhane (20 mL) est ajoutée le dendron 5 (1 équivalent, 0,8 mmol, Polymer Factory PFd-G3-Acetylene-OH) et une solution de 4-diméthylaminopyridine (0,2 équivalents/-OH, 1,3 mmol) dans la pyridine (5 équivalents/-OH, 32 mmol). La réaction évolue à 25°C pendant 24 heures. La phase organique est lavée avec une solution aqueuse de carbonate de sodium 10% et avec une solution saturée de chlorure de sodium, séchée sur sulfate de magnésium puis concentrée. Une chromatographie sur gel de silice (heptane/acétate d'éthyle) permet d'isoler la molécule 6 ou *D6* (0,3 mmol). Comme intermédiaire de synthèse, la molécule *7* est préparée à partir de la molécule 8 (1 équivalent, 0,5 mmol) en faisant réagir celle-ci avec de l'azide de sodium (1,3 équivalent, 0,65 mmol) dans du diméthylsulfoxyde (5 mL) par irradiation micro-ondes pendant 30 minutes à 120°C [9]. A une solution de la molécule 6 ou *D6* (1 équivalent, 0,3 mmol) dans un mélange tétrahydrofurane/eau 1/2 (10 mL) est additionnée 1,2 équivalents de la molécule 7 (0,4 mmol) en présence de 0,1 équivalents de CuSO₄ et de 0,3 équivalents d'ascorbate de sodium. La réaction procède à 25°C pendant 24 h. La molécule *D2* est ensuite purifiée par chromatographie sur gel de silice et est obtenue avec un rendement global de 12% (0,2 mmol).

Les spectres de résonance magnétique nucléaire (RMN) de proton et de carbon de la molécule *D2* sont exposés en figure 5.

### 1.3. Amorceur D3 :

### Synthèse de la molécule D3 :

La molécule *D2* (1 équivalent, 0,2 mmol) est laissée réagir avec 1 équivalent de la molécule 9 en présence de 0,2 équivalents de 4-diméthylaminopyridine et de 1,2 équivalents de N,N-dicyclohexylcarbodiimide dans du dichlorométhane (10 mL) pour former la molécule *D3* qui est purifiée par chromatographie sur colonne.

La molécule 9 est disponible dans le commerce.

La figure 9 montre la structure de l'amorceur de polymérisation utilisé dans cet exemple, la molécule *D3*, et sa voie de synthèse à partir de *D2*.

La figure 10 montre le spectre de résonance magnétique nucléaire de carbon de *D3* (spectre du bas). Le spectre de résonance magnétique nucléaire de carbon de *D2* avant couplage avec le composé 9 est également donné à titre comparatif (spectre du haut). Les flèches montrent bien que le signal correspondant à la fonction -COOH libre disparaît dans D3, signal qui est présent dans D2 avant couplage avec le composé 9.

### 1.4. Amorceur D4 :

Un autre exemple de réalisation d'un multiamorceur est la molécule *D4* dont la structure est décrite dans la figure 13. Cette molécule est basée sur une structure dendritique (dendron de génération 3), comprenant :
- au coeur une fonction (F2) qui est une fonction acétylène permettant une fixation par « chimie clic » (« click chemistry » en anglais) sur une autre entité comportant un groupement azide, et
- à la périphérie 8 fonctions (F1) qui sont des fonctions di(benzylester) du benzyl-N,N-di(carboxyméthyl)dithiocarbamate permettant de générer des radicaux réactifs, et donc d'amorcer une polymérisation radicalaire (amorceur de type iniferter pour polymérisation radicalaire vivante).

### Synthèse de la molécule D4 :

Les étapes de synthèse, les réactifs utilisés et la structure de la molécule *D4* obtenue sont illustrés en figure 13.

La molécule *1* (1 équivalent, 1 mmol, Sigma-Aldrich 530360) et la molécule *13* (1,2 équivalents, 1,2 mmol) sont dissous dans 20 mL de tétrahydrofurane (THF) sous atmosphère d'azote (La molécule 13 est obtenue à partir du N,N-di(carboxyméthyl) dithiocarbamate d'ammonium, préalablement synthétisé d'après G.R. Gale et al., Ann. Clin. Lab. Sci. 1983, 13, 474-481 [10], qui est ensuite estérifié avec l'alcool benzylique). La réaction évolue pendant 4 heures sous agitation à 70°C sous atmosphère d'azote. La phase organique est lavée par une solution saturée de chlorure de sodium, séchée sur sulfate de magnésium et concentrée. Une chromatographie sur gel de silice du résidu permet d'isoler le produit attendu *3*. La molécule *3* (0,9 mmol) est mise en réaction avec le N,N-dicyclohexylcarbodümide (1,5 équivalent, 1,8 mmol, Sigma-Aldrich 36650) dans le dichlorométhane (15 mL) à 25°C pendant 2 heures. Un précipité se forme et est éliminé par filtration sur fritté. Le filtrat est ensuite concentré à l'évaporateur rotatif pour donner l'anhydride *4*. A une solution de la molécule *10* (9,6 équivalents, 7,7 mmol, 1,5 équivalents/-OH) dans le dichlorométhane (20 mL) est ajoutée le dendron 5 (1 équivalent, 0,8 mmol, Polymer Factory PFd-G3-Acetylene-OH) et une solution de 4-diméthylaminopyridine (0,2 équivalents/-OH, 1,3 mmol) dans la pyridine (5 équivalents/-OH, 32 mmol). La réaction évolue à 25°C pendant 24 heures. La phase organique est lavée avec une solution aqueuse de carbonate de sodium 10% et avec une solution saturée de chlorure de sodium, séchée sur sulfate de magnésium puis concentrée. Une chromatographie sur gel de silice (heptane/acétate d'éthyle) permet d'isoler la molécule *D4* (0,3 mmol). La molécule D4 est ensuite purifiée par chromatographie sur gel de silice et est obtenue avec un rendement global de 13% (0,2 mmol).

A partir de la molécule *D4*, la molécule 11 peut être obtenue par hydrogénolyse catalytique des fonctions ester benzylique à la périphérie de D4.

### 1.5. Amorceur D5 :

Un exemple de réalisation d'un multiamorceur est la molécule *D5* décrite en figure 14. Cette molécule est basée sur une structure dendritique (dendron de génération 3), contenant dans le coeur une fonction (F2) qui est une fonction benzyl carboxylate (-COBz), et contenant sur sa périphérie des fonctions (F1) amorceurs de polymérisation radicalaire vivante de type iniferter (benzyldiéthyldithiocarbamate).

### Synthèse de la molécule D5 :

Les étapes de synthèse, les réactifs utilisés et la structure de la molécule *D5* obtenue sont illustrés dans la figure 14.

La molécule *1* (1 équivalent, 1 mmol, Sigma-Aldrich 530360) et la molécule 2 (1,2 équivalents, 1,2 mmol, Sigma-Aldrich 228680) sont dissous dans 20 mL de tétrahydrofurane (THF) sous atmosphère d'azote. La réaction évolue pendant 4 heures sous agitation à 70°C sous atmosphère d'azote. La phase organique est lavée par une solution saturée de chlorure de sodium, séchée sur sulfate de magnésium et concentrée. Une chromatographie sur gel de silice du résidu permet d'isoler le produit attendu 3. La molécule *3* (0,9 mmol) est mise en réaction avec le N,N-dicyclohexylcarbodiimide (1,5 équivalent, 1,8 mmol, Sigma-Aldrich 36650) dans le dichlorométhane (15 mL) à 25°C pendant 2 heures. Un précipité se forme et est éliminé par filtration sur fritté. Le filtrat est ensuite concentré à l'évaporateur rotatif pour donner l'anhydride *4*. En présence de 4-(diméthylamino)pyridine (0,2 équivalents/-OH, 1,3 mmol) et de pyridine (5 équivalents/-OH, 32 mmol), la molécule *4* (9,6 équivalents, 7,7 mmol, 1,5 équivalents/-OH) et le dendron *12* (1 équivalent, 0,8 mmol, Polymer Factory PFd-G3-COBz-OH) sont dissous dans le dichlorométhane (20 mL). La réaction évolue à 25°C pendant 48 heures pour donner la molécule *D5*, qui est purifié par chromatographie sur gel de silice (heptane/acétate d'éthyle 40/60), avec un rendement global de 40%.

Les spectres de résonance magnétique nucléaire (RMN) de proton et de carbon de la molécule *D5* sont exposés en figures 15A et 15B.

### Exemple 2 : Polymère à empreinte moléculaire (PEM) et procédé de préparation selon l'invention à partir de l'amorceur D2

Cet exemple décrit la synthèse d'un polymère à empreinte moléculaire (PEM) pour la molécule cible S-propranolol en utilisant l'amorceur D2. D2 est capable d'établir des liaisons avec le S-propranolol, ce dernier servant comme molécule empreinte. Le procédé est représenté de façon schématique dans la figure 3.

### Synthèse du PEM

**Tableau 1 : Formulations et réactifs**

| **Fonctions** | **Produits** | **Quantités employées** |
|---|---|---|
| **Molécule empreinte** | S-propranolol | 3,0 mg |
| **Monomère fixant** | aucun, l'amorceur agit comme monomère en même temps. | - |
| **Monomère réticulant** | Ethylène glycol diméthacrylate | 154,0 µL |
| **Amorceur** | D2 | 68,6 mg |
| **Solvant** | Acetonitrile | 3 mL |

On prépare une solution A en pesant 3,0 mg de S-propranolol que l'on dissout dans 1,5 mL d'acétonitrile puis on ajoute 154 µL d'éthylène glycol diméthacrylate. On place l'ensemble dans un bain à ultrason jusqu'à dissolution complète.

On prépare de même une solution B en pesant 68,6 mg de *D*2 que l'on dissout dans 1,5 mL d'acétonitrile.

On place à nouveau l'ensemble dans un bain à ultrason jusqu'à dissolution complète.

Dans le noir, on mélange la solution A avec la solution B dans un tube à essai en verre de 15 mL et on ferme le tube avec un bouchon à septum. On place le tube dans la glace pour 10 min puis on fait passer de l'argon dans la solution à travers le septum pendant 3 min.

On irradie le tube avec une lampe UV à 350 nm pendant un temps donné pour polymériser (de 5 min. à 12 heures). Les billes de polymère en suspension obtenues sont incubées à 25°C dans des mélanges méthanol/acide acétique 4:1 (3 fois pendant 1 h) et dans du méthanol (3 fois pendant 1 h). Ce traitement permet de libérer les empreintes du polymère à empreintes moléculaires obtenu.

L'évolution de la taille de particules de PEM est proportionnelle au logarithme de la dose lumineuse utilisée pour induire la photopolymérisation. Le diamètre peut être facilement modulé entre 30 nm et 3 µm en ajustant le temps d'exposition.

### Caractéristiques et propriétés du PEM

La figure 6 illustre un cliché de particules sphériques de PEM obtenus selon le procédé de l'invention, par microscopie électronique à transmission (cliché du haut). On peut observer qu'en utilisant un amorceur multifonctionnel selon l'invention, les particules formées sont denses et d'un diamètre de 1,3 µm environ. Dans la figure 6, le cliché du bas montre des particules de PEM obtenus par un procédé mettant en oeuvre un amorceur monofonctionnel, le benzyldiméthyldithiocarbamate, le nombre total de fonctions dithiocarbamate étant le même dans les deux cas. Il apparaît clairement que selon le procédé et en particulier l'amorceur utilisé, les PEM résultants n'ont pas la même taille, morphologie ou forme.

Pour évaluer l'effet impression moléculaire, le PEM et un polymère contrôle chimiquement identique mais synthétisé en absence de S-propranolol sont incubés avec le propranolol marqué au tritium (³H-S-propranolol). Pour cela, dans deux séries de tubes à essai de 1,5 mL sont introduits des quantités croissantes (de 100 à 900 µg) de PEM et du polymère contrôle, respectivement, suspendus dans de l'acétonitrile contenant 0,5% d'acide acétique. A cette suspension est rajouté 1 pmol de ³H-S-propranolol, puis les tubes sont incubés sous agitation pendant 2h à 25°C. Après centrifugation, la radioactivité dans le surnageant est déterminée par comptage de scintillation en phase liquide. La valeur obtenue correspond au ³H-S-propranolol non lié au polymère, et la fraction liée peut être calculée par la différence entre la quantité de ³H-S-propranolol retrouvé dans le surnageant et la quantité de ³H-S-propranolol totale rajoutée à chaque tube d'essai.

Les résultats obtenus sont représentés sur le graphe en figure 7. Il est clairement visible que le PEM adsorbe plus de ³H-S-propranolol que le polymère contrôle, donc il y a eu création de sites de reconnaissance par impression moléculaire.

### Exemple 3 : Polymère à empreinre moléculaire (PEM) et procédé de préparation selon l'invention à partir de l'amorceur D3

En analogie avec l'approche décrite dans la partie l'exemple, un PEM capable de reconnaître une protéine a été synthétisé en utilisant un amorceur dendritique couplé à une molécule d'ancrage permettant de former une interaction stable avec la protéine (figure 8).

Comme exemple non-limitant, la protéase trypsine a été choisie comme molécule empreinte, et un de ses inhibiteurs connus, la benzamidine, comme molécule d'ancrage. La benzamidine a été couplée au groupement COOH de l'amorceur dendritique *D2* donnant ainsi la molécule *D3.*

### Synthèse du PEM

**Tableau 2 : Synthèse et Formulation**

| **Fonctions** | **Produits** | **Quantités employées** |
|---|---|---|
| **Molécule empreinte** | trypsine | 17,2 mg |
| **Monomère fixant** | hydroxyéthyl méthacrylate (HEMA) | 54 mg |
| **Monomère réticulant** | éthylène bisacrylamide (EBA) | 128,7 mg |
| **Amorceur** | D3 | 2,7 mg |
| **Solvant** | tampon phosphate 20 mM pH 7,0 | 3,5 mL |

On prépare une solution A en pesant 17,2 mg de trypsine dans 500 µL de tampon phosphate 20 mM pH 7,0 puis on ajoute une solution de HEMA et EBA dans 3 mL de tampon phosphate 20 mM pH 7,0. On agite doucement l'ensemble jusqu'à dissolution complète.

Dans le noir, on prépare de même une solution B en pesant 2,7 mg de D3 dans DMSO (100 µL). On agite à nouveau l'ensemble jusqu'à dissolution complète.

Dans le noir, on mélange la solution A avec la solution B dans un tube à essai en verre de 15 mL et on ferme le tube avec un bouchon à septum, puis on agite doucement pendant 10 min a 4°C. On place le tube dans la glace pendant 10 min puis on fait passer de l'argon dans la solution à travers le septum pendant 3 min.

On irradie le tube avec une lampe UV à 350 nm pour un temps donné, à 4°C pour polymériser. Les billes de polymère en suspension obtenues sont incubées à 25°C dans une solution d'urée 2 M (4 fois pendant 1 h) afin de libérer les empreintes des molécules empreintes.

L'évolution de la taille de particules de PEM est proportionnelle au temps de photopolymérisation. Le diamètre peut être facilement modulé entre 10 nm et 3 µm en ajustant le temps d'exposition.

Afin d'évaluer le PEM et de confirmer l'effet impression, les particules sont incubées dans un tampon tris(hydroxyméthyl)aminométhane 5 mM à pH 8,0 avec une solution de trypsine à 0,6 µM dans un tampon tris(hydroxyméthyl)aminométhane 5 mM à pH 8,0 pendant 2 h à 4°C sous agitation. Après centrifugation pour sédimenter les particules, la concentration de trypsine dans le surnageant est déterminée en mesurant son activité catalytique à l'aide d'un spectrophotomètre double-faisceau UV-visible ou d'un spectrophotomètre de fluorescence en utilisant respectivement un substrat chromogénique (le chlorure de *N*_{α}-Benzoyl-L-arginine 4-nitroanilide) ou un substrat fluorogénique (le chlorure de Boc-Gln-Ala-Arg-7-amido-4-methylcoumarine).

On observe que le PEM adsorbe plus de trypsine que le polymère contrôle, donc il y a eu création de sites de reconnaissance par impression moléculaire.

### Exemple 4 : Polymère à empreinte moléculaire (PEM) associé à une surface solide et procédé de préparation selon l'invention à partir de l'amorceur D2

En analogie avec l'approche décrite dans l'exemple 2, un PEM directement associé à une surface de substrat solide a été synthétisé. L'exemple décrit la synthèse sur une surface de verre d'un PEM sélectif pour le beta-bloquant S-propranolol. La formulation employée dans cet exemple est similaire à celle utilisée dans l'exemple 2, avec la différence que la molécule amorceur D2 est immobilisée chimiquement à la surface d'un substrat solide avant polymérisation (figure 11).

Sur un substrat en verre sont successivement déposés une première couche de 2 nm d'épaisseur de chrome comme couche d'apprêt servant avantageusement à assister le dépôt d'une seconde couche de 47 nm d'épaisseur d'or. Les deux couches peuvent être déposées par toute technique connue usuelle pour l'homme du métier, préférentiellement par évaporation. La surface est ensuite incubée dans une solution de 5 mM de cystéamine dans l'éthanol pendant 8 h à 20°C, puis rincée à l'éthanol et séchée sous flux d'azote. Après la première incubation, la surface est de nouveau incubée dans une solution contenant l'amorceur *D2* (0,1 mmol), de l'hydroxybenzothiazole (0,1 mmol) et du N,N-diisopropylcarbodiimide (0,1 mmol) dans 3 mL DMF anhydre, sous agitation pendant 12 h à 20°C. La surface est ensuite rincée au DMF puis au méthanol, et séchée sous flux d'azote.

### Synthèse du PEM

**Tableau 3 : Formulations et réactifs**

| **Fonctions** | **Produits** | **Quantités employées** |
|---|---|---|
| **Molécule empreinte** | S-propranolol | 10 mg |
| **Monomère fixant** | Acide méthacrylique | 17,0 µL |
| **Monomère réticulant** | Ethylène glycol diméthacrylate | 145,0 µL |
| **Amorceur** | - | - |
| **Solvant** | Acetonitrile | 1,0 mL |

On prépare une solution en pesant 10 mg de S-propranolol que l'on dissout dans 1 mL d'acétonitrile puis on ajoute les 17 µL d'acide méthacrylique et les 145 µL d'éthylène glycol diméthacrylate. On place l'ensemble dans un bain à ultrason jusqu'à dissolution complète. On fait passer de l'argon dans le mélange à polymériser. Ensuite on dépose ce mélange sur le substrat revêtu d'or puis couvert par une lame de verre. La polymérisation est amorcée en irradiant le mélange réactionnel par une lampe UV 350 nm. Après un temps de polymérisation approprié (de quelques secondes à quelques minutes selon l'épaisseur désirée), un film fin de PEM est obtenu sur la surface d'or.

Variante 1 : A la place du substrat couvert d'une couche de chrome et d'or, un substrat en verre ou en silicium est utilisé qui est au préalable fonctionnalisé avec l'aminopropyl triméthoxysilane afin d'introduire des groupements amino pour le couplage de l'amorceur D2. La surface de verre est rincée à l'éthanol puis à l'eau milliQ. Elles est ensuite placée dans un bain d'ammoniac (teneur 4,6%) et portée pendant 5 minutes à 80°C. Puis, elle est placée dans un bain de peroxyde d'hydrogène (teneur 5%) pendant 5 minutes à 80°C. Puis, elle est placée dans une solution contenant du peroxyde d'hydrogène (teneur 4,3%) et de l'acide chlorhydrique (teneur 5,3%) à 80°C pendant 5. La surface de verre est ensuite rincée deux fois avec de l'eau milliQ, deux fois avec de l'acétone et deux fois avec du toluène. Elle est ensuite placée dans une solution contenant 50 mL de toluène et 1 mL d'aminopropyl triméthoxysilane pendant 12h à 20°C dans un récipient fermé. Ensuite, la surface est rincée trois fois avec de l'acétone puis séchée sous un flux d'azote. Le couplage de l'amorceur D2 se fait ensuite en incubant la surface dans une solution contenant D2 (0,1 mmol), de l'hydroxybenzothiazole (0,1 mmol) et du diisopropylcarbodiimide (0,1 mmol) dans 3 mL DMF anhydre, sous agitation pendant 12 h à 20°C. La surface est ensuite rincée au DMF puis au méthanol, et sechée sous flux d'azote.

Variante 2 : Au moment du couplage par le diisopropylcarbodiimide, un excès d'acide acétique (par rapport au nombre de moles de D2) est rajouté au mélange, résultant en un espacement des molécules amorceur greffées sur la surface du substrat. On utilise par exemple 0,099 mmol d'acide acétique et 0,001 mmol de *D2*. Le résultat est l'obtention d'un tapis de nano-ilôts plutôt qu'un film continu sur la surface.

### Exemple 5 : Polymère à empreinte moléculaire (PEM) et procédé de préparation selon l'invention à partir de l'amorceur D5

Cet exemple décrit la synthèse d'un polymère à empreinte(s) moléculaire(s) (PEM) pour la molécule cible S-propranolol en utilisant l'amorceur *D5*. Dans cet exemple, les concentrations de monomères sont choisies de façon à obtenir des billes de taille nanométriques de polymère à empreintes moléculaires.

### Synthèse du PEM

**Tableau 2 : Formulations et réactifs**

| **Fonctions** | **Produits** | **Quantités employées** |
|---|---|---|
| **Molécule empreinte** | S-propranolol | 12,0 mg |
| **Monomère fixant** | Acide méthacrylique | 31,2 µL |
| **Monomère réticulant** | Ethylène glycol diméthacrylate | 348,0 µL |
| **Amorceur** | D5 | 0,67 mg |
| **Solvant** | Acétonitrile | 2000 µL |

On prépare une solution A en pesant 12,0 mg de S-propranolol que l'on dissout dans 1 mL d'acétonitrile puis on ajoute 31,2 µM d'acide méthacrylique et 348 µL d'éthylène glycol diméthacrylate. On place l'ensemble dans un bain à ultrason jusqu'à dissolution complète.

On prépare de même une solution B en pesant 0,204 µmol de D5 que l'on dissout dans 1 mL d'acétonitrile.

On place à nouveau l'ensemble dans un bain à ultrason jusqu'à dissolution complète.

Dans le noir, on mélange la solution A avec la solution B dans un tube à essai en verre de 4 mL et on ferme le tube avec un bouchon à septum. On place le tube dans la glace pour 10 min puis on fait passer de l'argon dans la solution à travers le septum pendant 2 min.

On place le tube à 10°C et on l'irradie avec une lampe UV à 365 nm pendant 15 min pour polymériser. Les nanobilles de polymère en suspension obtenues sont incubées à 25°C dans des mélanges méthanol/acide acétique 4:1 (3 fois pendant 1 h) et dans du méthanol (3 fois pendant 1 h). Ce traitement permet de libérer les empreintes du polymère à empreintes moléculaires obtenu.

### Caractéristiques et propriétés du PEM

La figure 12 illustre un cliché de particules sphériques de PEM obtenus selon le procédé de l'invention, par microscopie électronique à transmission. On peut observer que les particules formées ont un diamètre de 50 nm environ. Dans la figure 12, le graphe du milieu montre l'analyse des particules de PEM par diffusion dynamique de lumière. On observe bien une taille moyenne de 45 nm et une faible polydispersité. Si la même synthèse est faite par un procédé identique mais mettant en oeuvre un amorceur monofonctionnel, le benzyl-N,N-diméthyldithiocarbamate, le nombre total de fonctions dithiocarbamate étant le même dans les deux cas, on n'obtient pas de particules de PEM après synthèse. Il apparaît clairement que l'utilisation de l'amorceur D5 permet d'obtenir des nanoparticules de PEM contrairement à l'amorceur monofonctionnel, le benzyl-N,N-diméthyl dithiocarbamate.

Pour évaluer l'effet impression moléculaire, le PEM et un polymère contrôle chimiquement identique mais synthétisé en absence de S-propranolol sont incubés avec le propranolol marqué au tritium (³H-S-propranolol). Pour cela, dans deux séries de tubes à essai de 1,5 mL sont introduits des quantités croissantes (de 100 à 4000 µg) de PEM et du polymère contrôle, respectivement, suspendus dans de l'acétonitrile. A cette suspension est rajouté 1 pmol de ³H-S-propranolol, puis les tubes sont incubés sous agitation pendant 2h à 25°C. Après centrifugation, la radioactivité dans le surnageant est déterminée par comptage de scintillation en phase liquide. La valeur obtenue correspond au ³H-S-propranolol non lié au polymère, et la fraction liée peut être calculée par la différence entre la quantité de ³H-S-propranolol retrouvé dans le surnageant et la quantité de ³H-S-propranolol totale rajoutée à chaque tube d'essai.

Les résultats obtenus sont représentés sur le graphe en bas de la figure 12. Il est clairement visible que le PEM adsorbe plus de ³H-S-propranolol que le polymère contrôle synthétisé sans la molécule empreinte S-propranolol, donc il y a eu création de sites de reconnaissance par impression moléculaire.

### Exemple 6 : Polymère à empreinte moléculaire (PEM) et procédé de préparation selon l'invention à partir de l'amorceur D5

Cet exemple décrit la synthèse d'un polymère à empreinte moléculaire (PEM) pour la molécule cible S-propranolol en utilisant l'amorceur *D5*. Dans cet exemple, les concentrations de monomères sont choisies de façon à obtenir des billes de taille nanométriques de polymère à empreintes moléculaires.

### Synthèse du PEM

**Tableau 2 : Formulations et réactifs**

| **Fonctions** | **Produits** | **Quantités employées** |
|---|---|---|
| **Molécule empreinte** | S-propranolol | 12,0 mg |
| **Monomère fixant** | Acide méthacrylique | 31,2 µL |
| **Monomère réticulant** | Ethylène glycol diméthacrylate | 348,0 µL |
| **Amorceur** | D5 | 6,7 mg |
| **Solvant** | Acetonitrile | 10,5 mL |

On prépare une solution A en pesant 12,0 mg de S-propranolol que l'on dissout dans 5 mL d'acétonitrile puis on ajoute 31,2 µM d'acide méthacrylique et 348 µL d'éthylène glycol diméthacrylate. On place l'ensemble dans un bain à ultrason jusqu'à dissolution complète.

On prépare de même une solution B en pesant 2,04 µmol de D5 que l'on dissout dans 5,5 mL d'acétonitrile.

On place à nouveau l'ensemble dans un bain à ultrason jusqu'à dissolution complète.

Dans le noir, on mélange la solution A avec la solution B dans un tube à essai en verre de 15 mL et on ferme le tube avec un bouchon à septum. On place le tube dans la glace pour 10 min puis on fait passer de l'argon dans la solution à travers le septum pendant 2 min.

On place le tube à 30°C et on l'irradie avec une lampe UV à 365 nm pendant 16h pour polymériser.

On obtient un rendement de polymérisation, ici exprimé en % conversion des monomères, de 21%. Si la même synthèse est faite par un procédé identique mais mettant en oeuvre un amorceur monofonctionnel, le benzyl-N,N-diméthyldithiocarbamate, le nombre total de fonctions dithiocarbamate étant le même dans les deux cas, le rendement de polymérisation n'est que de 5%. Il apparaît clairement que l'utilisation de l'amorceur D5 permet d'obtenir des nanoparticules de PEM avec un rendement supérieur comparé avec l'amorceur monofonctionnel, le benzyl-N,N-diméthyldithiocarbamate.

### Listes des références

[1] K. Mosbach, O.Ramström, Bio/Technology 1996, 14, 163-170.
[2] B. Rückert, A. Hall, B.Sellergren, J. Mater. Chem. 2002, 12, 2275-2280.
[3] A. Biffis, N. B. Graham, G. Siedlaczek, S. Stalberg, G. Wulff, Macromol. Chem. Phys. 2001, 202, 163-171.
[4] M. Antonietti, C. Rosenauer, Macromolecules 1991, 24, 3434 -3442.
[5] G. Wulff, B.-O. Chong, U. Kolb, Angew. Chem. Int. Ed. 2006, 45, 2955 -2958.
[6] T. Otsu, J. Polym. Sci.: Part A: Polym. Chem. 2000, 38, 2121-2136.
[7] R. C. Mehrotra, "Present status and future potential of the Sol-Gel process", dans Chemistry, Spectroscopy and Applications of Sol-Gel Glasses, Vol. 77 de la série Structure and Bonding, Springer, Berlin/Heidelberg, 1992.
[8] R. H. Schmidt, K. Haupt (2005), Chem. Mater. 17, 1007-1016.
[9] Y. Ju, D. Kumar, R. S. Varma, J. Org. Chem., 2006, 71, 6697-6700.
[10] G.R. Gale GR, L.M. Atkins, E.M. Walker Jr, A.B. Smith, M.M. Jones, Ann. Clin. Lab. Sci. 1983, 13, 474-481.
[11] H.C. Kolb, K.B. Sharpless, Drug Discov. Today, 2003, 8, 1128-1137.
[12] H.C. Kolb, M.G. Finn, K.B. Sharpless, K.B. Angew. Chem. InEd., 2001, 40, 2004-2021.

## Revendications

1. Procédé de préparation d'un polymère à empreinte(s) moléculaire(s) (PEM) par polymérisation radicalaire dans lequel :
a) on met en contact :
▪ au moins une entité ou molécule empreinte ;
▪ des monomères identiques ou différents, capables de former le polymère à empreinte(s) moléculaire(s) (PEM) ;
▪ au moins un amorceur comportant au moins deux fonctions chimiques (F1) capables de former des radicaux permettant d'amorcer la polymérisation desdits monomères sur ou autour de ladite entité ou molécule empreinte ;
b) on effectue la polymérisation sous l'action d'un rayonnement lumineux de longueur d'onde visible ou UV ou sous l'action d'un apport d'énergie thermique formant ainsi sur ou autour de ladite entité ou molécule empreinte un polymère à empreinte(s) moléculaire(s) (PEM) ;
c) on élimine le ou les monomères non polymérisés et/ou tout ou partie de l'entité ou molécule empreinte du polymère formé en b) avec un solvant ou un mélange de solvant(s) approprié(s).

2. Procédé selon la revendication 1, dans lequel l'entité ou molécule empreinte est choisie dans le groupe comprenant:
- les aminoacides, les oses, les lipides, les nucléosides, les nucléotides, les oligomères et polymères obtenus à partir de ces entités ou molécules, notamment les peptides, protéines, acides nucléiques, oligosaccharides et polysaccharides,
- les entités ou molécules biologiquement actives, notamment les médicaments, notamment les antibiotiques, les hormones stéroïdes, les vitamines, les toxines, les enzymes, les agents de dopage, les pesticides, insecticides, fongicides et herbicides,
- les substances explosives,
- les substances toxiques,
- les perturbateurs endocriniens,
- les nanoparticules,
- les cellules de bactéries procaryotes et d'eucaryotes
- les cellules animales, notamment les cellules de levures, les cellules de champignons et les cellules animales et humaines
- les cellules de plantes,
- les virus, et
- les tissus cellulaires.

3. Procédé selon l'une des revendication 1 ou 2, dans lequel l'entité ou molécule empreinte est choisie dans le groupe comprenant la trypsine, la kallicréine, le S-propranolol, la théophylline, l'acide 2,4-dichlorophénoxyacétique, le beta-estradiol, le testosterone, l'atrazine, la morphine, la cocaïne, le tétrahydrocannabinol.

4. Procédé selon la revendication 1, dans lequel dans l'étape a), outre l'entité ou molécule empreinte, on ajoute un substrat.

5. Procédé selon la revendication 4, dans lequel le substrat est choisi dans le groupe comprenant le verre, le quartz, le mica, le silicium, le germanium, le carbure de silicium, l'oxyde d'étain-indium, le dioxyde de titane, l'oxyde d'aluminium, un polymère organique éventuellement réticulé notamment le polystyrène, le poly(styrène-co-méthacrylate), le poly(méthylméthacrylate), le poly(acrylonitrile), un polyamide, un polyester, un polyuréthane, le poly(diméthylsiloxane), le polybutadiène, ou un métal notamment l'or, l'argent, le platine, ou un composite de deux ou plusieurs de ces matériaux.

6. Procédé selon la revendication 1, dans lequel les monomères sont des monomères fixants éventuellement en mélange avec des monomères réticulants.

7. Procédé selon la revendications 6, dans lequel les monomères comportent au moins une fonction vinyle choisi dans le groupe comprenant :
- l'acide méthacrylique, l'acide trifluorométhylacrylique, l'acide acrylique, l'acide itaconique, l'acide styrènesulfonique, l'acide acrylamidopropanesulfonique, l'acide 4-vinylphénylboronique, l'acide 4-vinylbenzoique, le carboxyéthylacrylate, l'hydroxyéthylméthacrylate phosphate, le 2-vinylpyridine, le 4-vinylpyridine, le 1-vinylimidazole, le 4(5)-vinylimidazole, le 2-aminoéthylméthacrylate, le 2-(diméthylamino)éthyl méthacrylate, l'aminoéthylacrylate, le chlorure de 4-vinylbenzyltriméthylammonium, l'hydroxyéthylméthacrylate, le glycidylméthacrylate, l'acrylamide, le méthacrylamide, l'isopropylacrylamide, le 4-vinylbenzamidine, le 4-méthacrylamidobenzamidine, le styrène, le phénylméthacrylate, le cyclohexylméthacrylate, le méthylméthacrylate, le butylméthacrylate, le hexylméthacrylate, le octylméthacrylate, l'éthylène glycol diméthacrylate, l'hexanediol diméthacrylate, le trisméthylolpropane triméthacrylate, le polyéthylène glycol diméthacrylate, le triéthylène glycoldiméthacrylate, le pentaérythritol triacrylate, le divinylbenzène, l'éthylène bisacrylamide, le méthylène bisacrylamide, le bisacryloyl pipérazine, le N,N-bisacryloylcystamine, le *N,N*-1,2-dihydroxyéthylène bisacrylamide, le N,O-bisméthacryloyl éthanolamine ;
- ou un mélange de ceux-ci.

8. Procédé selon l'une quelconque des revendications 1 à 7, dans lequel l'amorceur comporte jusqu'à 64 fonctions chimiques (F1) capables de former des radicaux permettant d'amorcer la polymérisation des monomères sur ou autour ladite entité ou molécule empreinte.

9. Procédé selon l'une quelconque des revendications 1 à 8, dans lequel les fonctions chimiques de l'amorceur capable de former des radicaux permettant d'amorcer la polymérisation sont apportées par un composé choisis dans le groupe comprenant :
- les éthers de benzoïne notamment le méthyléther de benzoïne, l'éthyléther de benzoïne, le butyléther de benzoïne, l'isopropyléther de benzoïne, les acétophénones notamment la 2,2-diméthoxy-2-phényl acétophénone, le 2-hydroxy-2-méthyl-1-phényl-propan-1-one ou le 2-méthyl-1-[4-(méthylthio)phényl]-2-morpholinopropan-1-one, les benzophénones substitués ;
- les peroxydes substitués notamment le bis(tert-butyl cyclohexyl)peroxydicarbonate, les dérivés des phosphine-oxydes notamment le bis(2,4,6-triméthylbenzoyl)-phénylphosphine-oxyde, les amino-cétones, les oxysulfonyl-cétones, les sulfonyl-cétones ou l'un de ces dérivés, et les composés de type azo notamment l'azo-bis-isobutyronitrile, l'azo-bis-dimethylvaléronitrile, l'acide 4,4'-azobis-cyanovalérique, le 2,2'-azobis(2-amidinopropane) ;
- les hydroxy-alkylamines notamment la méthyldiéthanolamine, les benzylamines, les dérivés d'aniline, le paradiméthylaminobenzoate d'éthyle, la N-phénylglycine (NPG) ;
- l'acide ascorbique ;
- un dérivé de dithiocarbamate notamment le benzyl-N,N-diméthyldithio-carbamate, le benzyl-N,N-diéthyldithiocarbamate, le benzyl-N,N-di(2-hydroxyéthyl)dithio-carbamate, le benzyl-N-méthyl-N-(2hydroxyéthyl)dithiocarbamate, le benzyl-N-méthyl-N-carboxyméthyldithiocarbamate, le di(benzylester) du benzyl-N,N-di di(carboxyméthyl)dithiocarbamate

10. Procédé selon l'une quelconque des revendications 1 à 8, dans lequel l'amorceur comporte au moins une seconde fonction chimique (F2) choisie dans le groupe comprenant l'hydroxyle, l'acide carboxylique, l'ester carboxylique, le thioester, l'amide, l'amine, le thiol, l'éther, l'amidine, la guanidine, l'urée, la thiourée, l'aldéhyde, la cétone, l'alkyle, le phényle, le benzyle, l'acide iminodiacétique, l'acide nitrilotriacétique, l'acide éthylène diaminotétraacétique, un chélate métallique notamment l'acide iminodiacétique-Cu, l'acide iminodiacétique-Co, l'acide iminodiacétique-Ni, l'acide iminodiacétique-Zn, l'acide nitrilotriacétique-Ni, l'acétylène.

11. Procédé selon l'une quelconque des revendications 1 à 10, dans lequel dans l'étape a), l'amorceur est en mélange avec un composé sensibilisateur choisi dans le groupe comprenant :
- les acridines notamment l'Acriflavine (marque déposée) ou l'Acridine Orange (marque déposée), les phénazines notamment la Safranine O (marque déposée), les oxazines, les thiazines notamment le Bleu de Méthylène ou la thionine, les xanthènes notamment l'Eosine Y (marque déposée), le Rose Bengale (marque déposée) ou l'Erythrosine (marque déposée), les rhodamines, les thioxanthènes, les polyméthines ;
- les dérivés de la thioxanthone notamment l'isopropylthioxanthone ou le chlorothioxanthone, les couramines ou leurs dérivés notamment les cétocoumarines.

12. Procédé selon la revendication 1, dans lequel, dans l'étape a), la mise en contact est effectuée en présence d'au moins un solvant porogène choisi dans le groupe comprenant :
- le benzène, le toluène, le xylène, les solvants chlorés notamment le chloroforme, le dichlorométhane, le dichloroéthane et le 1,1,2,2,-tétrachloroéthane, et/ou les solvants perfluorés, notamment le hexafluorocyclohexane, les éthers notamment le tétrahydrofurane, le diéthylène glycol diméthyle éther et le triéthylène glycol diméthyle éther, les amides notamment le diméthylformamide et le formamide, les nitriles notamment l'acétonitrile et le caprylonitrile, les cétones notamment l'acétone, les esters notamment l'acétate d'éthyle, les alcanes notamment l'hexane et le heptane, les alcools notamment le méthanol, l'éthanol, l'iso-propanol, le butanol, le décanol, l'eau ;
- ou un mélange de ceux-ci.

13. Procédé selon la revendication 1, dans lequel, dans l'étape b) on effectue la polymérisation sous l'action d'un rayonnement lumineux de longueur d'onde visible ou UV d'une longueur d'onde comprise entre 100 nm et 1200 nm.

14. Procédé selon la revendication 1, dans lequel, dans l'étape b) on effectue la polymérisation sous un apport d'énergie thermique à une température comprise entre 0°C et 200°C.

15. Procédé selon la revendication 1, dans lequel, dans l'étape c), le solvant est choisi dans le groupe comprenant l'eau, le méthanol, l'éthanol, l'iso-propanol, l'acétonitrile, le chloroforme, l'heptane, le toluène, l'acide acétique, ou leur mélange ou le CO₂ supercritique.

16. Procédé selon la revendication 1, dans lequel, dans l'étape c), outre le solvant ou le mélange de solvants, on élimine l'entité ou molécule empreinte du polymère formé en b) avec un solvant ou un mélange de solvants contenant un autre agent choisi dans le groupe comprenant :
- un détergent notamment le dodecylsulfate the sodium, le bromure de cetyl triméthylammonium, le Triton X-100 (marque déposée), l'Empigen BB (marque déposée), ;
- l'urée ;
- l'acide trifluoroacétique ;
- la triéthylamine ;
- une enzyme hydrolytique choisie dans le groupe comprenant une peptidase ou une protéase, notamment la trypsine ou la protéinase K ;
- une nucléase, notamment la nucléase de *Staphylococcus aureus* ou la ribonucléase A ;
- une lipase, notamment la lipase du pancréas de porc ou la lipase XIII de *Pseudomonas sp. ;*
- une glycosidase, notamment la beta-glucosidase, la alpha-glucosidase, ou la beta-galactosidase ;
- un sel notamment le thiosulfate de sodium.

17. Procédé selon la revendication 1, dans lequel, dans l'étape c), outre le solvant, on élimine l'entité ou molécule empreinte du polymère formé en b) en appliquant un champs électrique.

18. Polymère à empreinte(s) moléculaire(s) (PEM) susceptible d'être obtenu par le procédé tel que défini à l'une quelconque des revendications 1 à 17.

19. Polymère selon la revendication 18 se présentant sous la forme d'un film d'une épaisseur comprise entre 1 nm et 100 µm.

20. Polymère selon la revendication 18 se présentant sous la forme de particules sphériques de diamètre compris entre 1 nm et 100 µm.

21. Polymère selon la revendication 18 ayant une structure poreuse dont le diamètre des pores est compris entre 0,1 nm et 10 µm.

22. Utilisation d'un polymère selon l'une quelconque des revendications 16 à 21, dans la réalisation biocapteurs biomimétiques, des biopuces biomimétiques, des capteurs chimiques, des dispositifs de séparation par adsorption spécifique, en tant que revêtement notamment pour le relargage de produits actifs.

23. Amorceurs de formules suivantes : 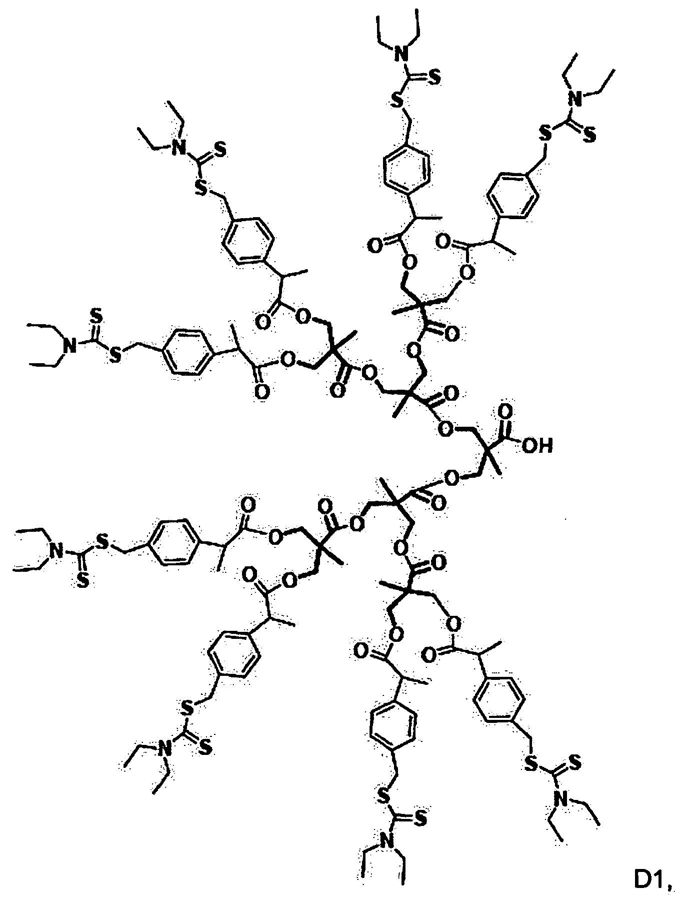 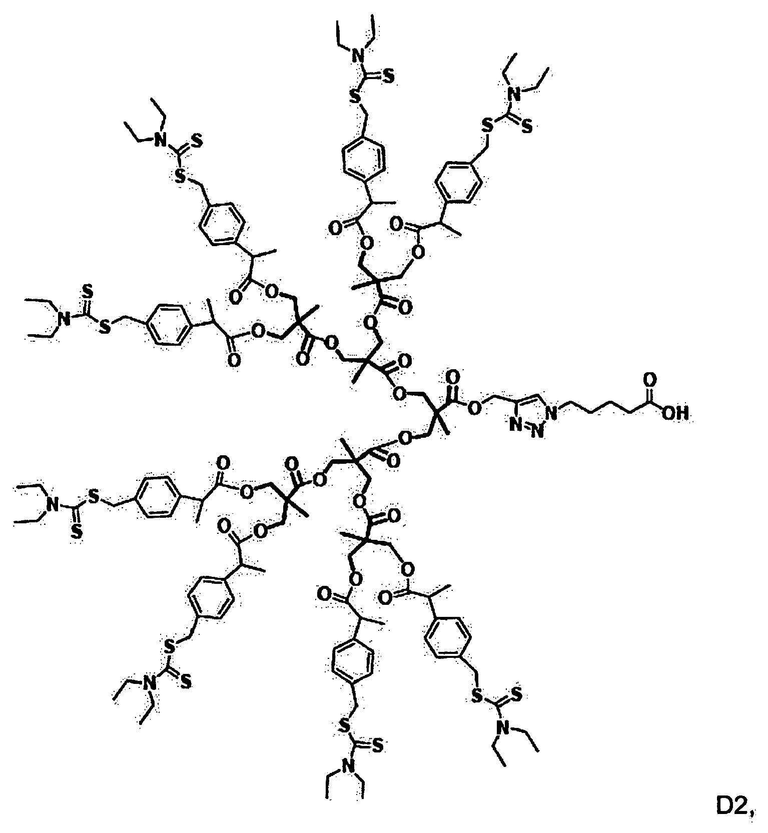 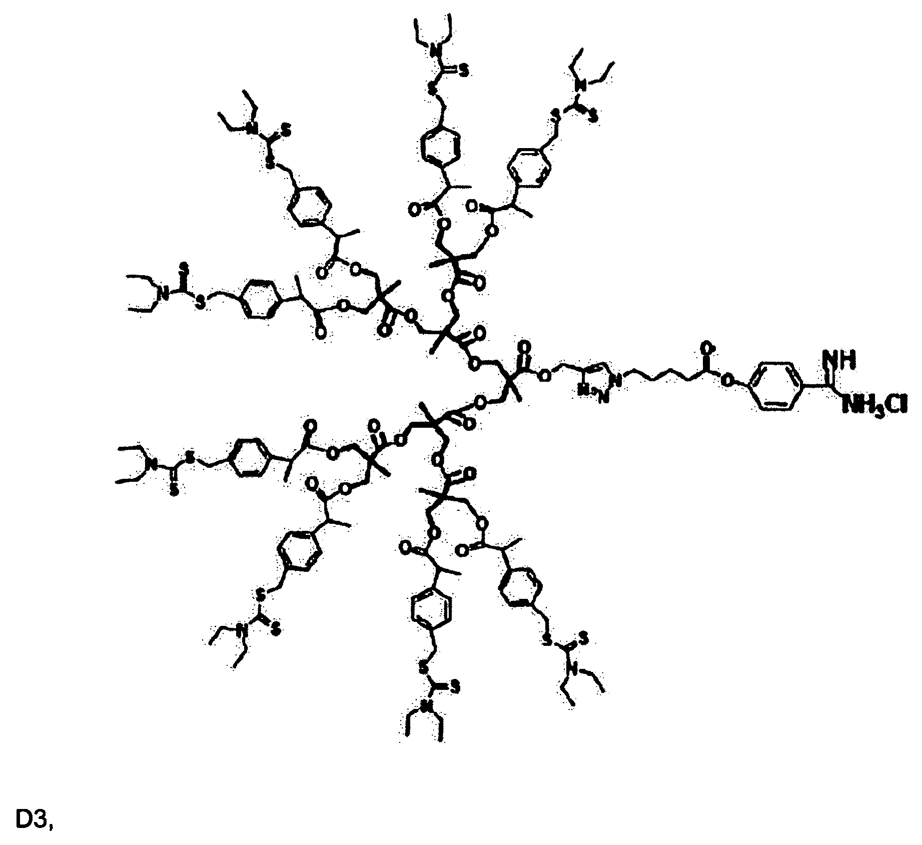 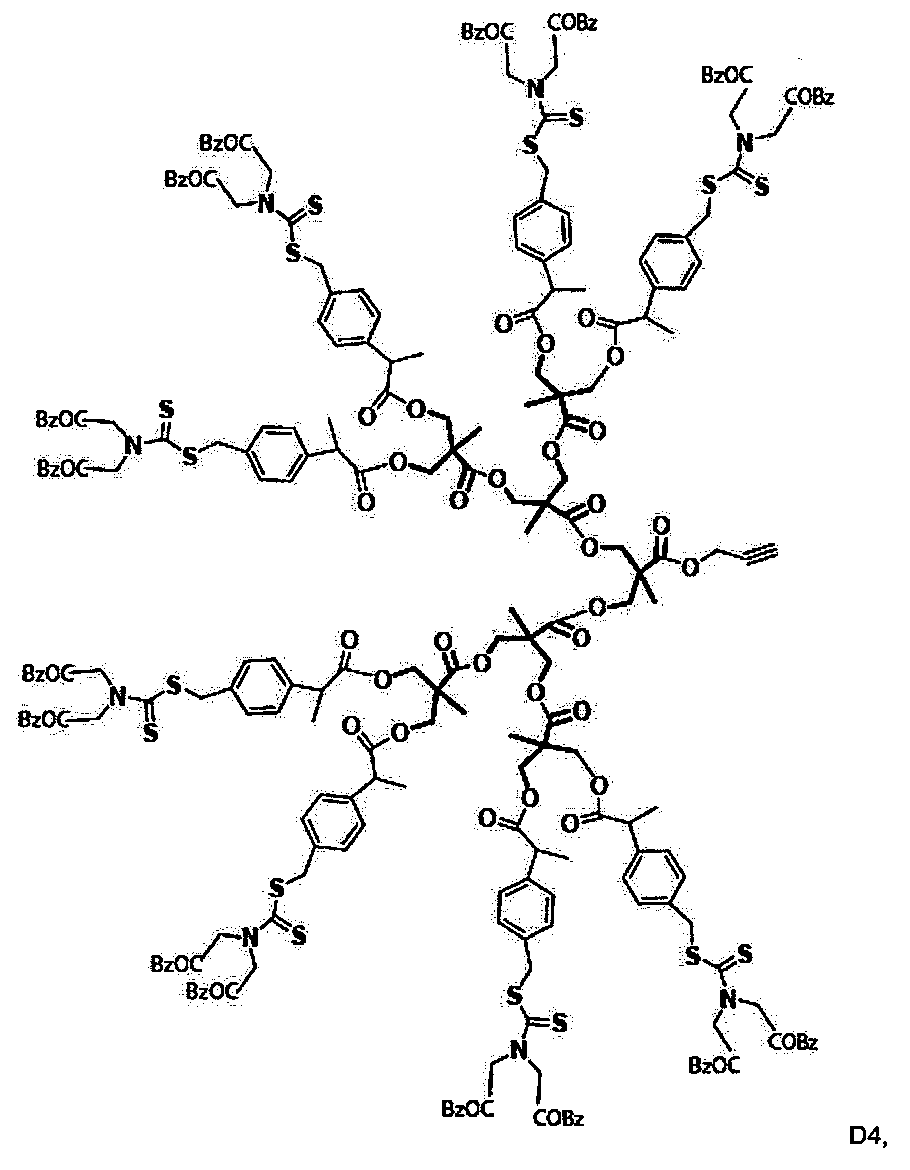 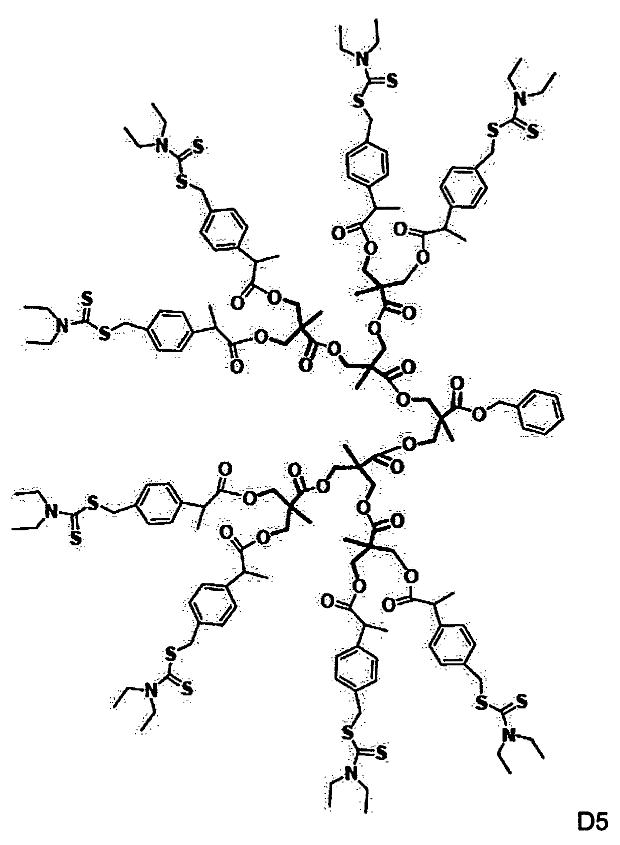 et

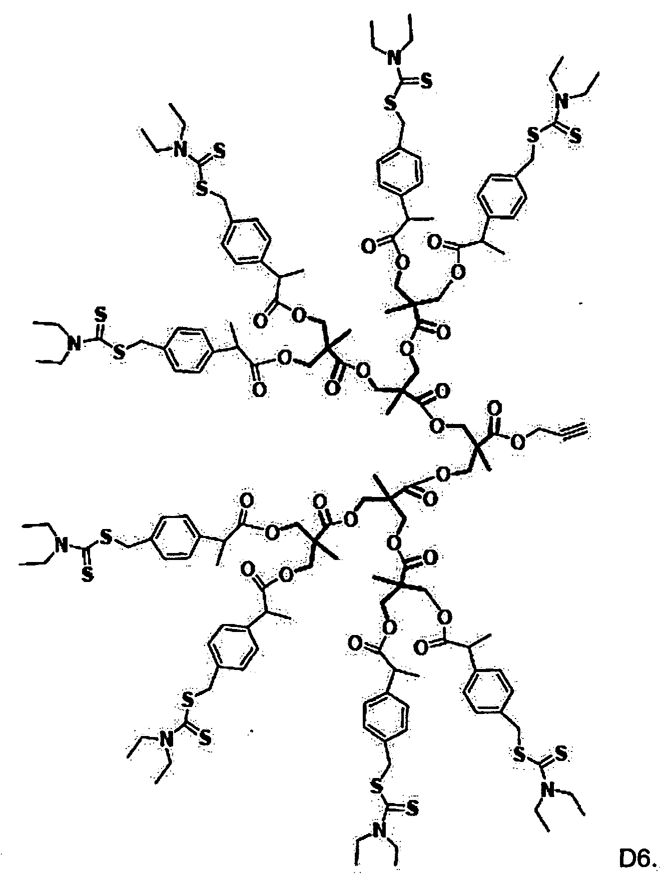

24. Utilisation d'un amorceur selon la revendication 23 pour la préparation d'un polymère à empreinte(s) moléculaire(s) par polymérisation radicalaire.

## Patentansprüche

1. Verfahren zur Vorbereitung eines Polymers mit molekularer Prägung/molekularen Prägungen (PMP) durch radikalische Polymerisation, wobei:
a) Folgendes miteinander in Kontakt gebracht wird:
- mindestens eine Einheit oder ein Prägemolekül;
- identische oder verschiedene Monomere, die das Polymer mit molekularer Prägung/molekularen Prägungen (PMP) bilden können;
- mindestens ein Initiator mit mindestens zwei chemischen Funktionen (F1), die Radikale bilden können, die ermöglichen, die Polymerisation der Monomere auf der oder um die Einheit oder dem/das Prägemolekül einzuleiten;
b) die Polymerisation unter der Einwirkung eines Lichtstrahls mit einer sichtbaren oder UV-Wellenlänge oder durch die Einwirkung einer Zuführung von thermischer Energie durchgeführt wird, die so auf der oder um die Einheit oder dem/das Prägemolekül ein Polymer mit molekularerr Prägung/molekularen Prägungen (PMP) bildet;
c) das oder die nicht polymerisierten Monomer(e) und/oder die Gesamtheit oder ein Teil der Einheit oder des Prägemoleküls des Polymers, gebildet in b), mit einem Lösemittel oder einer Mischung eines geeigneten/geeigneter Lösesmittel(s) entfernt werden.

2. Verfahren nach Anspruch 1, wobei die Einheit oder das Prägemolekül ausgewählt wird aus der Gruppe, umfassend:
- Aminosäuren, Osen, Lipide, Nukleoside, Nukleotide, Oligomere und Polymere, die auf der Grundlage dieser Einheiten oder Moleküle erhalten werden, insbesondere Peptide, Proteine, Nukleinsäuren, Oligosaccharide und Polysaccharide.
- biologisch aktive Einheiten oder Moleküle, insbesondere Medikamente, insbesondere Antibiotika, Sterioidhormone, Vitamine, Toxine, Enzyme, Dopingwirkstoffe, Pestizide, Insektizide, Fungizide und Herbizide,
- explosive Substanzen,
- toxische Substanzen,
- endokrine Disruptoren,
- Nanopartikel,
- prokaryontische und eukaryontische Bakterienzellen
- tierische Zellen, insbesondere Hefezellen, Pilzzellen und tierische und menschliche Zellen
- pflanzliche Zellen,
- Viren, und
- Zellgewebe.

3. Verfahren nach einem der Ansprüche 1 oder 2, wobei die Einheit oder das Prägemolekül ausgewählt ist aus der Gruppe, umfassend: Trypsin, Kallikrein, S-Propranolol, Theophyllin, 2,4-Dichlorphenoxyessigsäure, Beta-Estradiol, Testosteron, Atrazin, Morphin, Kokain, Tetrahydrocannabinol.

4. Verfahren nach Anspruch 1, wobei in Schritt a) ausser der Einheit oder dem Prägemolekül ein Substrat zugefügt wird.

5. Verfahren nach Anspruch 4, wobei das Substrat ausgewählt ist aus der Gruppe, umfassend: Glas, Quarz, Glimmer, Silizium, Germanium, Siliziumkarbid, Zinn-Indium-Oxid, Titandioxid, Aluminiumoxid, ein organisches Polymer gegebenenfalls vernetzt, insbesondere Polystyrol, Poly(styrol-co-methylmethacrylat), Poly(methylmethacrylat), Poly(acrylonitril), ein Polyamid, ein Polyester, ein Polyurethan, Poly(dimethylsiloxan), Polybutadien, oder ein Metall, insbesondere Gold, Silber, Platin, oder ein Verbundstoff von zwei oder mehreren dieser Materialien.

6. Verfahren nach Anspruch 1, wobei die Monomere fixierende Monomere sind, eventuell in Mischung mit vernetzenden Monomeren.

7. Verfahren nach Anspruch 6, wobei die Monomere mindestens eine Vinylfunktion umfassen, ausgewählt aus der Gruppe, umfassend:
- Methacrylsäure, Trifluoromethylacrylsäure, Acrylsäure, Itaconsäure, Styrolsulfonsäure, Acrylamidopropansulfonsäure, 4-Vinylphenylboronsäure, 4-Vinylbenzoesäure, Carboxy-Ethylacrylat, Hydroxyethylmethacrylat-Phosphat, 2-Vinylpyridin, 4-Vinylpyridin, 1-Vinylimidazol, 4(5)-Vinylimidazol, 2-Aminoethylmethacrylat, 2-(Dimethylamino)EthylMethacrylat, Aminoethylacrylat, 4-Vinylbenzyltrimethyl-Ammonium-Chlorid, Hydroxyethylmethacrylat, Glycidylmethacrylat, Acrylamid, Methacrylamid, Isopropylacrylamid, 4-Vinylbenzamidin, 4-Methacrylamidobenzamidin, Styrol, Phenylmethacrylat, Cyclohexylmethacrylat, Methylmethacrylat, Butylmethacrylat, Hexylmethacrylat, Octylmethacrylat, Ethylenglykoldimethacrylat, Hexanedioldimethacrylat, Trimethylolpropantrimethacrylat, Polyethylenglykoldimethacrylat, Triethylenglykoldimethacrylat, Pentaerythritoltriacrylat, Divinylbenzol, Bisacrylamidethylen, Bisacrylamidmethylen, Bisacryloylpiperazin, N,N-Bisacryloylcystamin, *N,N*-1,2-Dihydroxyethylen-Bisacrylamid, N,O-Bismethacryloyl-Ethanolamin;
- oder eine Mischung dieser.

8. Verfahren nach einem der Ansprüche 1 bis 7, wobei der Initiator bis zu 64 chemische Funktionen (F1) umfasst, die dazu in der Lage sind, Radikale zu bilden, die ermöglichen, die Polymerisation der Monomere auf der oder um die Einheit oder dem/das Prägemolekül zu initiieren.

9. Verfahren nach einem der Ansprüche 1 bis 8, wobei die chemischen Funktionen des Initiators, die dazu in der Lage sind, Radikale zu bilden, die ermöglichen, die Polymerisation zu initiieren, durch eine Verbindung zugegeben werden, ausgewählt aus der Gruppe, umfassend:
- Benzoinether, insbesondere Benzoinmethylether, Benoinethylether Benzoinisopropylether, Acetophenone, insbesondere 2,2-Dimethoxy-2-phenylacetophenon, 2-Hydroxy-2-methyl-1-phenylpropan-1-on oder 2-Methyl-1-[4-(methylthio)phenyl]-2-morpholinopropan-1-on, substituierte Benzophenone;
- substituierte Peroxide, insbesondere bis(tert-Butyl-cyclohexyl)-peroxydikarbonat, Derivate der Phosphinoxide, insbesondere bis(2,4,6-Trimethylbenzoyl)-phenylphosphinoxid, Aminoketone, Oxysulfonylketone, Sulfonylketone oder eines dieser Derivate, und die Verbindungen vom Typ Azo, insbesondere Azo-bis-Iobutyronitril, Azo-bisdimethylvaleronitril, 4,4'-Azobis-Cyanovaleriansäure, 2,2'-Azobis(2-Amidinopropan) ;
- Hydroxyalkylamine, insbesondere Methyldiethanolamin, Benzylamine, Anilinderivate, Paradimethylaminobenzoatethyl, N-Phenylglycin (NPG) ;
- Ascorbinsäure;
- ein Derivat von Dithiocarbamat, insbesondere Benzyl-N,N-Dimethyldithioccarbamat, Benzyl-N,N-Diethyldithiocarbamat, Benzyl-N,N-di(2-hydroxyethyl)dithiocarbamat, Benzyl-N-methyl-N-(2hydroxyethyl)-dithiocarbamat, Benzyl-N-methyl-N-Carboxymethyldithiocarbamat, Di(benzylester) des Benzyl-N,N-di di(carboxymethyl)dithiocarbamats

10. Verfahren nach einem der Ansprüche 1 bis 8, wobei der Initiator zumindest eine zweite chemische Funktionen (F2) umfasst, ausgewählt aus der Gruppe, umfassend: Hydroxyl, Carbonsäure, Carbonsäureester, Thioester, Amid, Amin, Thiol, Ether, Amidin, Guanidin, Harnstoff, Thioharnstoff, Aldehyd, Keton, Alkyl, Phenyl, Benzyl, Iminodiessigsäure, Nitrilotriessigsäure, Ethylendiamintetraessigsäure, ein Metallchelat, insbesondere Iminodiessigsäure-Cu, Iminodiessigsäure-Co, Iminodiessigsäure-Ni, Iminodiessigsäure-Zn, Iminodiessigsäure-Ni, Acetylen.

11. Verfahren nach einem der Ansprüche 1 bis 10, wobei in Schritt a) der Initiator in Mischung mit einer Sensibilisierungverbindung vorliegt, ausgewählt aus der Gruppe, umfassend: :
- Acridine, insbesondere Acriflavin (Warenzeichen) oder Acridineorange (Warenzeichen), Phenazine, insbesondere Safranin O (Warenzeichen), Oxazine, Thiazine, insbesondere Methylenblau oder Thionin, Xanthene, insbesondere Eosin Y (Warenzeichen), Rose Bengale (Warenzeichen) oder Erythrosin (Warenzeichen), Rhodamine, Thioxanthene, Polymethine;
- Drivate von Thioxanthon, insbesondere Isopropylthioxanthon oder Chlorothioxanthon, Curamine oder ihre Derivate, insbesondere Cetocumarine.

12. Verfahren nach Anspruch 1, wobei in Schritt a) das In-Kontakt-Bringen in Anwesenheit von mindestens einem porenbildenden Lösemittel durchgeführt wird, ausgewählt aus der Gruppe, umfassend:
- Benzol, Toluol, Xylen, chlorhaltige Lösemittel, insbesondere Chloroform, Dichloromethan, Dichloroethan und 1,1,2,2,-Tetrachloroethan, und/oder perfluorierte Lösemittel, insbesondere Hexafluorocyclohexan, Ether, insbesondere Tetrahydrofuran, Diethylenglycoldimethylether und Triethylenglycoldimethylether, Amide, insbesondere Dimethylformamid und Formamid, Nitrile, insbesondere Acetonitril und Caprylonitril, Ketone, insbesondere Azeton, Ester, insbesondere Ethylacetat, Alkane, insbesondere Hexan und Heptan, Alkohole, insbesondere Methanol, Ethanol, Iso-Propanol, Butanol, Decanol, Wasser;
- oder eine Mischung dieser.

13. Verfahren nach Anspruch 1, wobei in Schritt b) die Polymerisation unter der Einwirkung eines Lichtstrahls mit einer sichtbaren oder UV-Wellenlänge mit einer Wellenlänge zwischen 100 nm und 1200 nm durchgeführt wird.

14. Verfahren nach Anspruch 1, wobei in Schritt b) die Polymerisation unter Zuführung von thermischer Energie mit einer Temperatur zwischen 0 °C und 200 °C durchgeführt wird.

15. Verfahren nach Anspruch 1, wobei in Schritt c) das Lösemittel ausgewählt ist aus der Gruppe, umfassend: Wasser, Methanol, Ethanol, Iso-Propanol, Acetonitril, Chloroform, Heptan, Toluen, Essigsäure oder ihre Mischung oder superkritisches CO₂.

16. Verfahren nach Anspruch 1, wobei in Schritt c) ausser dem Lösemittel oder der Mischung aus Lösemitteln die Einheit oder das Prägemolekül des Polymers, gebildet in b), mit einem Lösemittel oder einer Mischung aus Lösemitteln entfernt wird, enthaltend ein weiteres Mittel, ausgewählt aus der Gruppe, umfassend:
- ein Waschmittel, insbesondere Natriumdodecylsulfat, Cetyltrimethylammoniumbromid, Triton X-100 (Warenzeichen), Empigen BB (Warenzeichen);
- Harnstoff;
- Trifluoressigsäure;
- Triethylamin;
- ein hydrolytisches Enzym, ausgewählt aus der Gruppe, umfassend eine Peptidase oder eine Protease, insbesondere Trypsin oder Proteinase-K;
- einer Nuklease, insbesondere *Staphylococcus aureus*-Nuklease oder Ribonuklease-A;
- eine Lipase, insbesondere Schweinepankreaslipase oder Lipase XIII von *Pseudomonas sp.;*
- eine Glycosidase, insbesondere Beta-Glucosidase, Alpha-Glucosidase oder Beta-Galactosidase;
- ein Salz, insbesondere Natriumthiosulfat.

17. Verfahren nach Anspruch 1, wobei in Schritt c) ausser dem Lösemittel die Einheit oder das Prägemolekül des Polymers, gebildet in Schitt b), entfernt wird, indem ein elektrisches Felde angewendet wird.

18. Polymer mit molekularer Prägung/molekularen Prägungen (PMP), das durch das Verfahren erhalten werden kann, wie in einem der Ansprüche 1 bis 17 definiert.

19. Polymer nach Anspruch 18, das die Form eines Films mit einer Dicke zwischen 1 nm und 100 µm aufweist

20. Polymer nach Anspruch 18, das die Form von sphärischen Partikeln mit einem Durchmesser zwischen 1 nm und 100 µm aufweist

21. Polymer nach Anspruch 18 mit einer porösen Struktur, wobei der Durchmesser der Poren zwischen 0,1 nm und 10 µm liegt

22. Verwendung eines Polymers nach einem der Ansprüche 16 bis 21 bei der Herrstellung von biomimetischen Biosensoren, biomimetischen Biochips, chemische Sensoren, Vorrichtungen zur Trennung durch spezifische Adsorption, als Beschichtung, insbesondere zur Freisetzung von aktiven Produkten.

23. Initiatoren mit den folgenden Formeln: 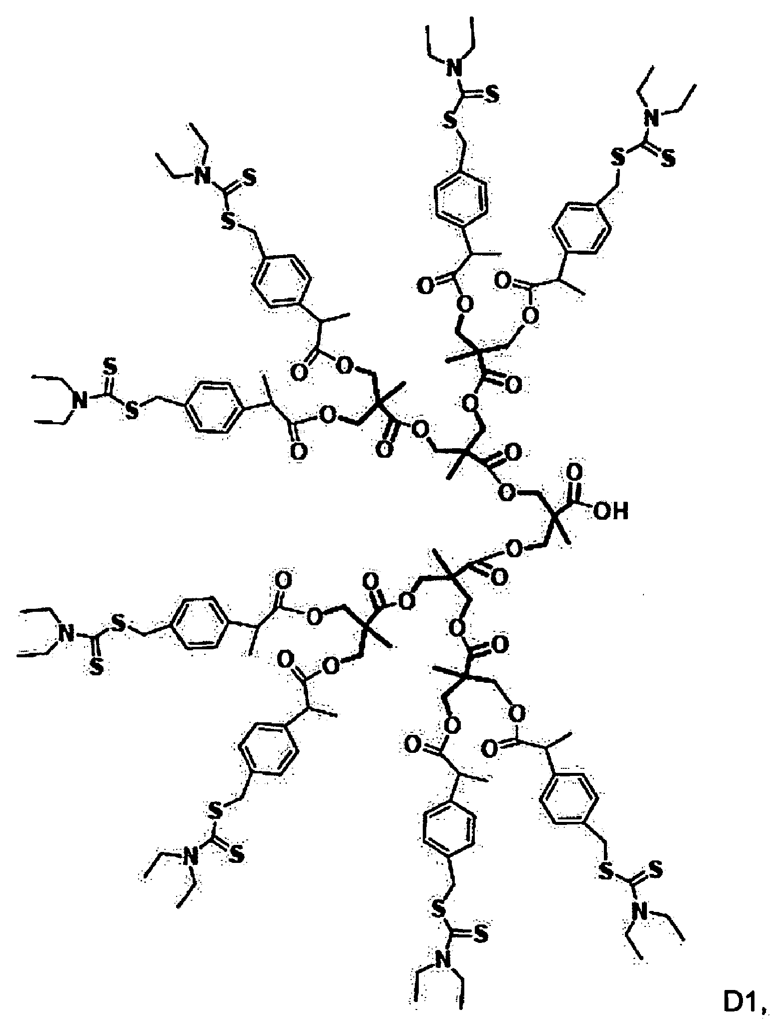 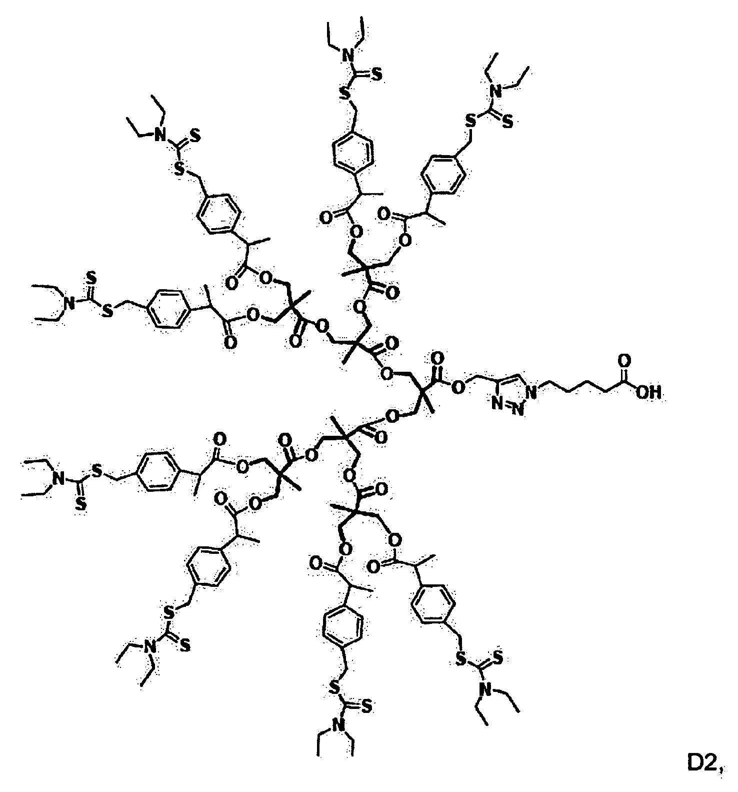 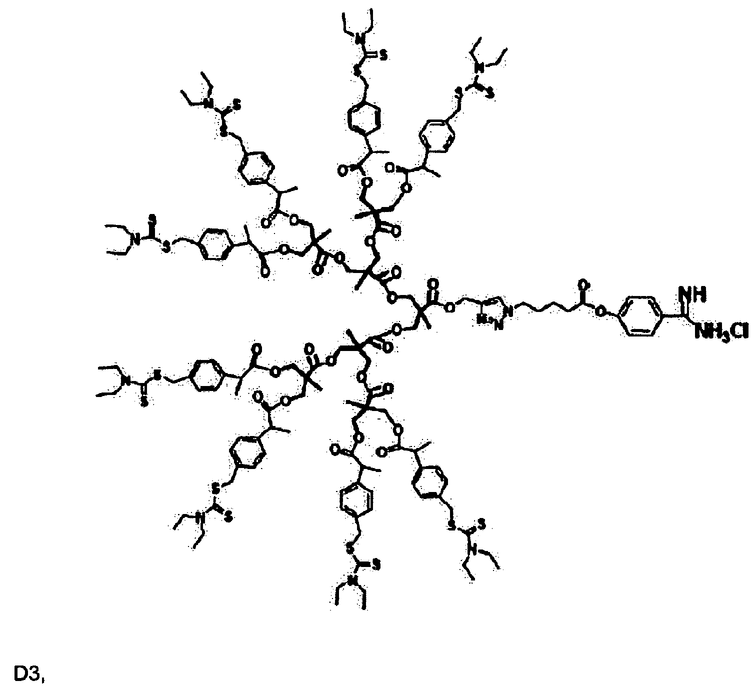 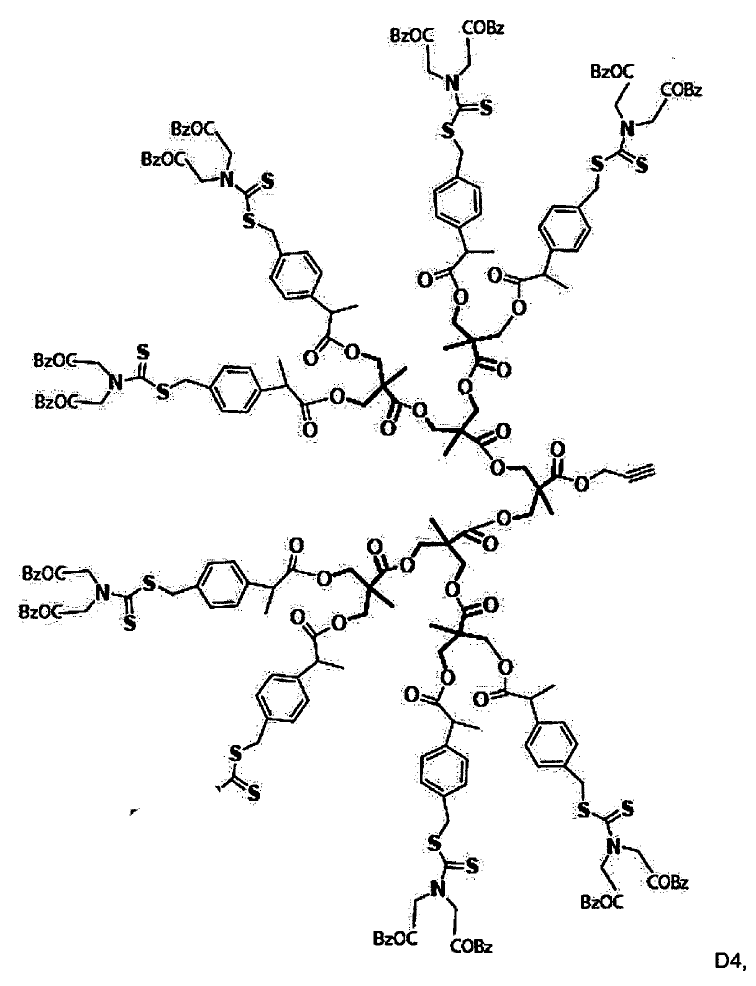 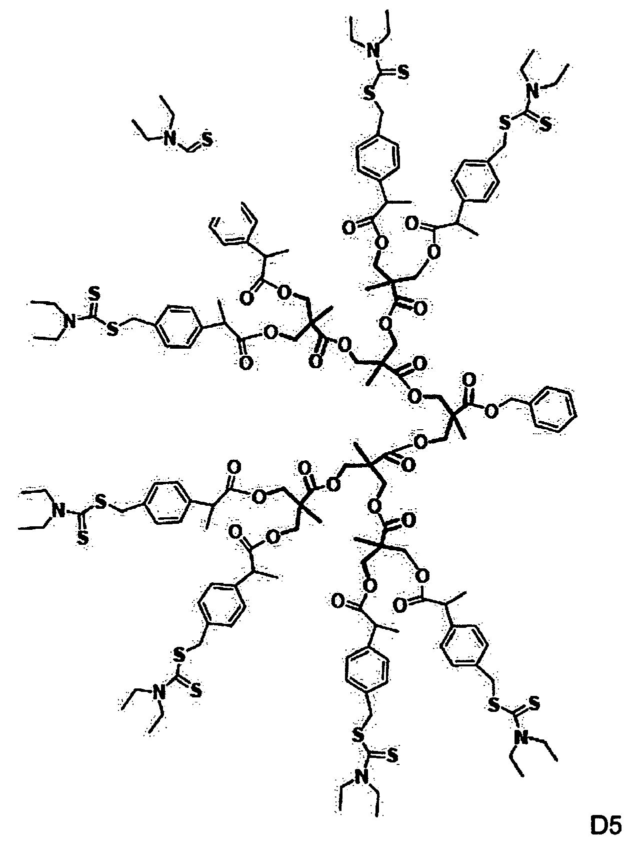 und

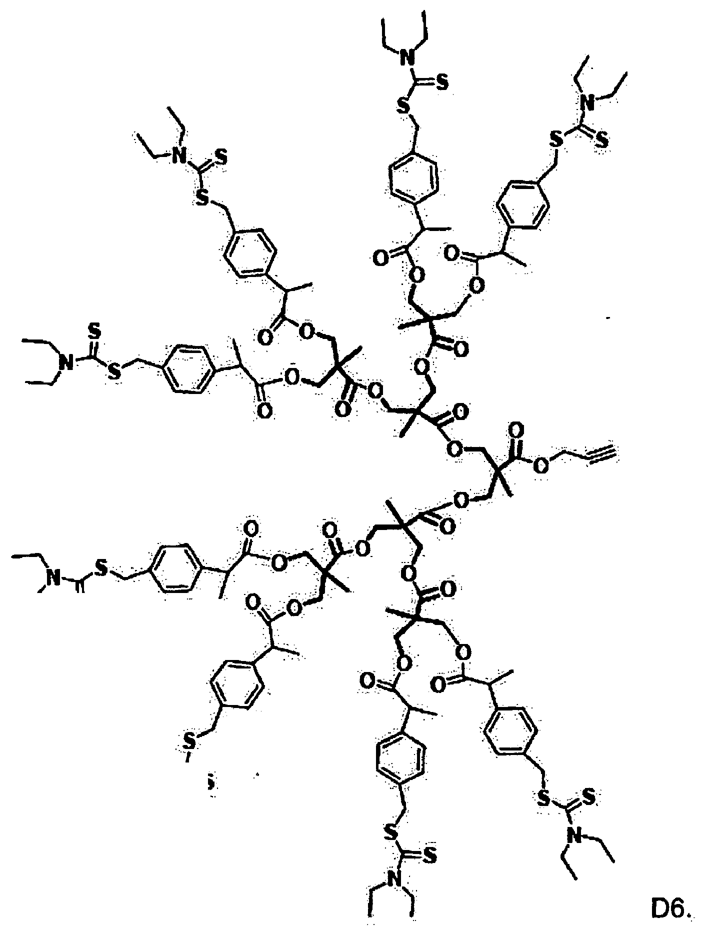

24. Verwendung eines Initiators nach Anspruch 23 zur Vorbereitung eines Polymers mit molekularer Prägung/molekularen Prägungen durch radikalische Polymerisation.

## Claims

1. A method for preparing a molecularly imprinted polymer (MIP) through radical polymerization, wherein :
a) are contacted with each other :
- at least one imprinting molecule or entity ;
- identical or different monomers, capable of forming the molecularly imprinted polymer (MIP) ;
- at least one initiator including at least two chemical functionality (F1) capable of forming radicals for initiating the polymerization of said monomers over or around said imprinting molecule or entity ;
b) a polymerization under exposure to light irradiation of visible or UV wavelength or with supply of thermal energy is performed thereby forming over or around said imprinting molecule or entity a molecularly imprinted polymer (MIP) ;
c) non polymerized monomer or monomers and/or all or part of the imprinting molecule or entity of the polymer formed in b) are removed with a solvent or a convenient mixture of solvent or solvents.

2. The method according to claim 1, wherein the imprinting molecule or entity is selected from the group comprising :
- amino acids, monosaccharide, lipids, nucleosides, nucleotides, oligomers and polymers obtained from entities or molecules, in particular, peptides, proteins, nucleic acids, oligosaccharides and polysaccharides;
- biologically active entities or molecules, in particular drugs, particularly, antibiotics, steroids, vitamins, toxins, enzymes, doping agents, pesticides, insecticides, fungicides and herbicides,
- explosive substances,
- toxic substances,
- endoctrine perturbators,
- nanoparticles,
- prokaryotic and eukaryotic bacteria cells,
- animal cells, in particular, yeast cells, fungus cells, and human and animal cells
- plant cells,
- viruses, and
- cellular tissues.

3. The method according to anyone of claim 1 or 2, wherein the imprinting molecule or entity is selected from the group comprising typsine, kallikrein, S-propranolol, theophyllin, 2,4-dichlorophenoxyacetic acid, beta-estradiol, testosterone, atrazine, morphine, cocaine, tetradydrocannabinol.

4. The method according to claim 1, wherein in step a), in addition to the imprinting molecule or entity, a substrate is added.

5. The method according to claim 4, wherein the substrate is selected from the group comprising glass, quartz, mica, silicon, germanium, silicon carbide, tin-indium oxide, titanium dioxide, aluminum oxide, an organic polymer which may be cross-linked, particularly, polystyrene, poly(styrene-co-methacrylate), poly(methyl methacrylate), poly(acrylonitrile), polyamide, polyester, polyurethane, poly(dimethyl siloxane), polybutadien, or a metal, particularly, gold, silver, platinum, or a composite of two or more of these materials.

6. The method according to claim 1, wherein the monomers are monomers which may bond when mixed with crosslinking monomers.

7. The method according to claim 6, wherein the monomers include at least a vinyl functionality selected from the group comprising:
- methacrylic acid, trifluoromethylacrylic acid, acrylic acid, itaconic acid, styrenesulfonic acid, acrylamidopropanesulfonic acid, 4-vinylphenolyl boronic acid, 4-vinylbenzoic acid, carboxyethylacrylate, hydroxyethyl methacrylate phosphate, 2-vinylpyridine, 4-vinylpyridine, 1-vinylimidazole, 4(5)-vinylimidazole, 2-aminoethyl methacrylate, 2-(dimethylamino)ethyl methacrylate, aminoethyl acrylate, 4-vinylbenzyl trimethylammonium chloride, hydroxyethyl methacrylate, glycidyl methacrylate, acrylamide, methacrylamide, isopropylacrylamide, 4-vinylbenzamidine, 4-methacrylamidobenzamidine, styrene, phenyl methacrylate, cyclohexyl methacrylate, methyl methacrylate, butyl methacrylate, hexyl methacrylate, octyl methacrylate, ethylene glycol dimethacrylate, hexanediol dimethacrylate, tris-methylolpropane trimethacrylate, polyethylene glycol dimethacrylate, triethylene glycol dimethacrylate, pentaerythritol triacrylate, divinylbenzene, ethylene bisacrylamide, methylene bisacrylamide, bisacryloyl piperazine, N,N-bisacryloylcystamine, N,N-1,2-dihydroxyethylene bisacrylamide, N,O-bismethacryloyl ethanolamine ;
- or a mixture thereof.

8. The method according to any one of claims 1 to 7, wherein the initiator comprises up to 64 chemical functionalities (F1) capable of forming radicals for initiating polymerization of the monomers on or around said imprinting molecule or entity.

9. The method according to any one of claims 1 to 8, wherein the chemical functionalities (F1) of the initiator capable of forming radicals for initiating polymerization are brought through a compound selected from the group comprising:
- benzoin ethers, particularly, benzoin methylether, benzoin ehtylether, benzoin butylehter, benzoin isopropylether, acetophenones, particularly, 2,2-dimethoxy-2-phenylacetophenone, 2-hydroxy-2-methyl-1-phenyl-propan-1-one or 2-methyl-1-[4-(methylthio)phenyl]-2-morpholinopropan-1-one, substituted benzophenones;
- substituted peroxides, particularly, bis(tert-butyl cyclohexyl)peroxydicarbonate, phosphine oxides derivatives, particularly, bis(2,4,6-trimethylbenzoyl)-phenylphosphine-oxide, aminoketones, sulfonyloxy ketones, sulfonyl ketones or a derivative thereof, and azo-compounds particularly azo-bis-isobutyronitrile, azo-bis-dimethylvaleronitrile, 4,4'-azobis-cyanovaleric acid, 2,2'-azobis(2-amidinopropane) ;
- hydroxyalkylamines, particularly methylethanolamine, benzylamines, aniline derivatives, ethyl-para-(dimethylamino)benzoate, N-phenylglycine (NPG) ;
- ascorbic acid;
- a dithiocarbamate derivative particularly benzyl-N,N-dimethyldithio-carbamate, benzyl-N,N-diethyldithiocarbamate, benzyl-N,N-di(carboxymethyl)dithiocarbamate, benzyl-N,N-di(2-hydroxyethyl)dithio-carbamate, benzyl-N-methyl-N-(2-hydroxyethyl)dithiocarbamate, benzyl-N-methyl-N-carboxymethyldithiocarbamate, benzyl-N,N-di(carboxymethyl)dithiocarbamate di(benzylester).

10. The method according to any one of claims 1 to 8, wherein the initiator comprises at least a second chemical functionality (F2) selected from the group comprising hydroxyl, carboxylic acid, carboxylic ester, thioester, amide, amine, thiol, ether, amidine, guanidine, urea, thiourea, aldehyde, ketone, alkyl, phenyl, benzyl, iminodiacetic acid, nitrilotriacetic acid, ethylenediaminotetraacetic acid, a metallic chelate particularly, Cu-iminodiacetic acid, Co-iminodiacetic acid, Ni-iminodiacetic acid, Zn-iminodiacetic acid, Ni-nitrilotriacetic acid, acetylene.

11. The method according to any one of claims 1 to 10, wherein in step a), the initiator is mixed with a sensitizer compound selected from the group comprising:
- acridines particularly Acriflavine^{®} or Acridine Orange^{®}, phenazines particularly Safranine O^{®}, oxazines, thiazines, particularly methylene blue or thionine, xanthenes, particularly Eosine Y^{®}, Rose Bengal^{®} or Erythrosyne^{®}, rhodamines, thioxanthenes, polymethines;
- thioxanthone derivatives particularly isopropylthioxanthone or chlorothioxanthone, couramines or derivatives thereof, particularly keto-coumarines.

12. The method according to claim 1, wherein, in step a), the contact is performed in presence of at least a propogenic solvent selected from the group comprising:
- benzene, toluene, xylene, chlorinated solvents, particularly chloroform, dichloromethane, dichloroethane, 1,1,2,2-tetrachloro ethane, and/or perfluorated solvents, particularly hexafluorocyclohexane, ethers, particularly, tetrahydrofuran, diethylene glycol dimethyl ether, and triethylene glycol dimethyl ether, amides particularly dimethylformamide and formamide, nitriles particularly acetonitrile and caprylonitrile, ketones particularly acetone, esters particularly ethyl acetate, alkanes, particularly hexane and heptanes, alcohols particularly methanol, ethanol, isopropanol, butanol, decanol, water;
- or a mixture thereof.

13. The method according to claim 1, wherein, in step b), the polymerization is performed under exposure to light irradiation of visible wavelength or UV wavelength ranging between 100 nm and 1200 nm.

14. The method according to claim 1, wherein, in step b) the polymerization is performed with supply of thermal energy at a temperature comprised between 0°C and 200°C.

15. The method according to claim 1, wherein, in step c), the solvent is selected from the group comprising water, methanol, ethanol, isopropanol, acetonitrile, chloroform, heptanes, toluene, acetic acid, or a mixture thereof or supercritical CO₂.

16. The method according to claim 1, wherein in step c), in addition to the solvent or to the solvent mixture, the imprinting molecule or entity is removed from the polymer formed in b) by a solvent or solvent mixture containing another agent selected from the group comprising:
- a detergent particularly sodium dodecylsulfate, cetyltrimethylammonium bromide, Triton X-100^{®}, Empigen BB^{®};
- urea;
- trifluoroacetic acid;
- triethylamine;
- an hydrolytic enzyme selected from the group comprising a peptidase or a protease, particularly trypsin or proteinase K;
- nuclease, particularly *Staphylococcus aureus* nuclease or ribonuclease A;
- a lipase particularly porcine pancreatic lipase or *Pseudomonas sp.* Lipase XIII;
- glycosidase, particularly beta-glucosidase, alpha-glucosidase, or beta-galactosidase;
- a salt particularly sodium thiosulfate.

17. The method according to claim 1, wherein in step c), in addition to the solvent, the imprinting molecule or entity is removed from the polymer formed in b) by applying an electrical field.

18. A molecularly imprinted polymer (MIP) obtainable through the method according to any one of claims 1 to 17.

19. The polymer according to claim 18 in the form of a film the thickness of which is comprised between 1 nm and 100 µm.

20. The polymer according to claim 18 in the form of spherical particles the diameter of which is comprised between 1 nm and 100 µm.

21. The polymer according to claim 18 having a porous structure whose pore diameter is comprised between 0.1 nm and 10 µm.

22. Use of a polymer according to any one of claims 16 to 21, for making biomimetic biosensors, biomimetic biochips, chemical sensors, specific adsorption separation devices, as a coating, in particular, for salting out active products.

23. Initiators of following formula: 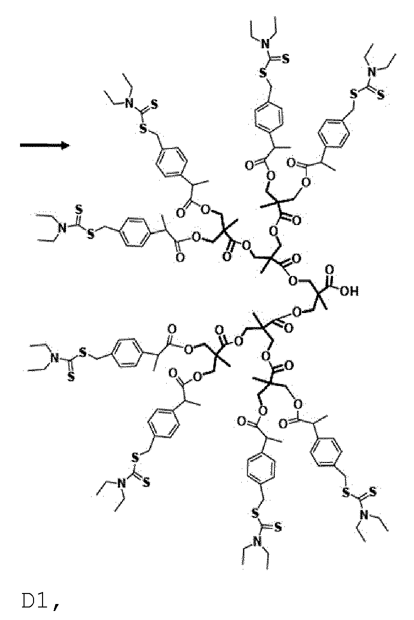 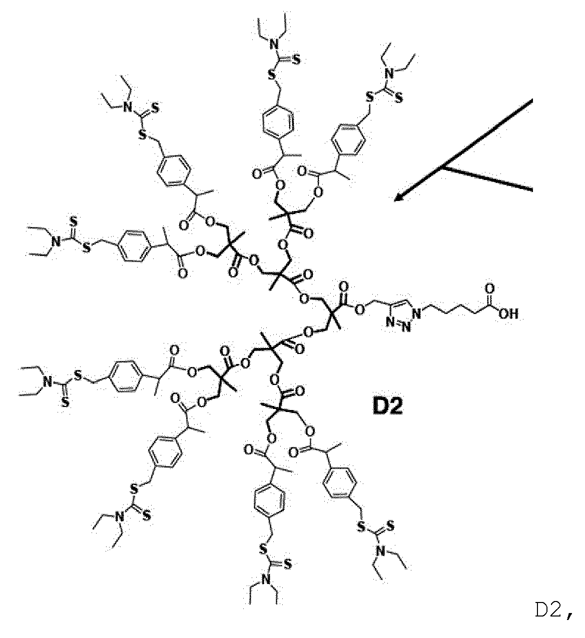 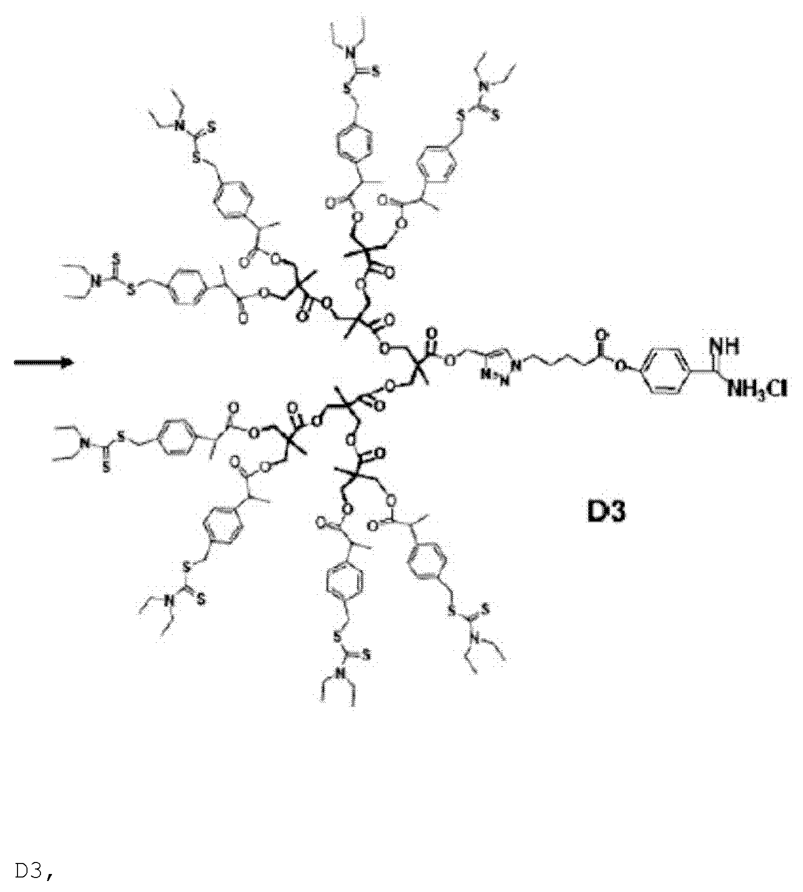 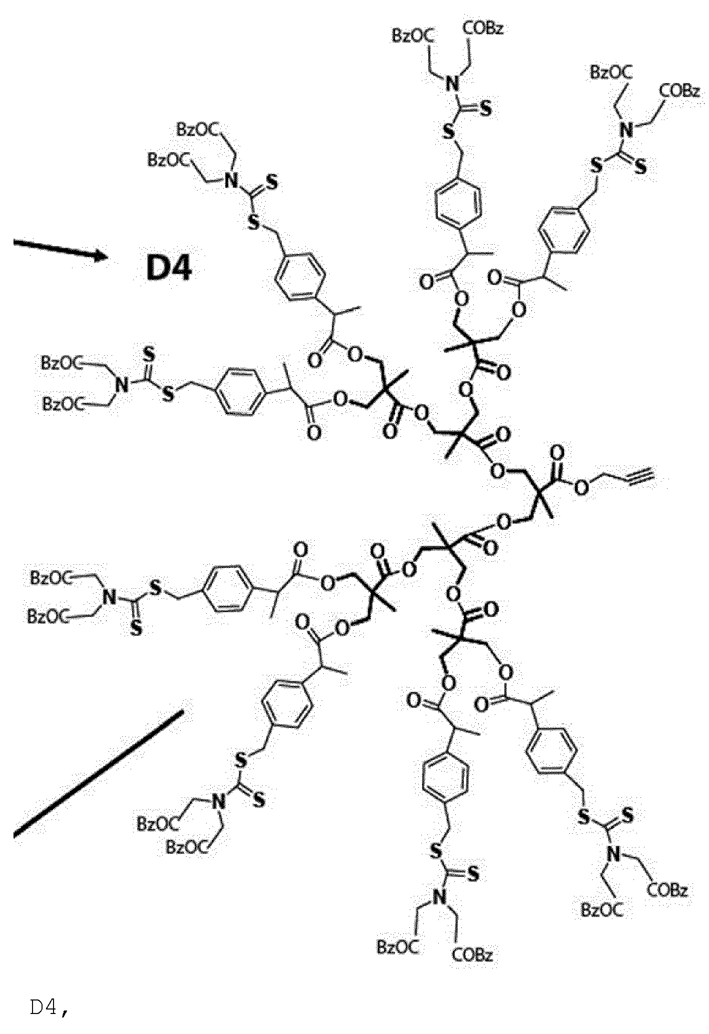 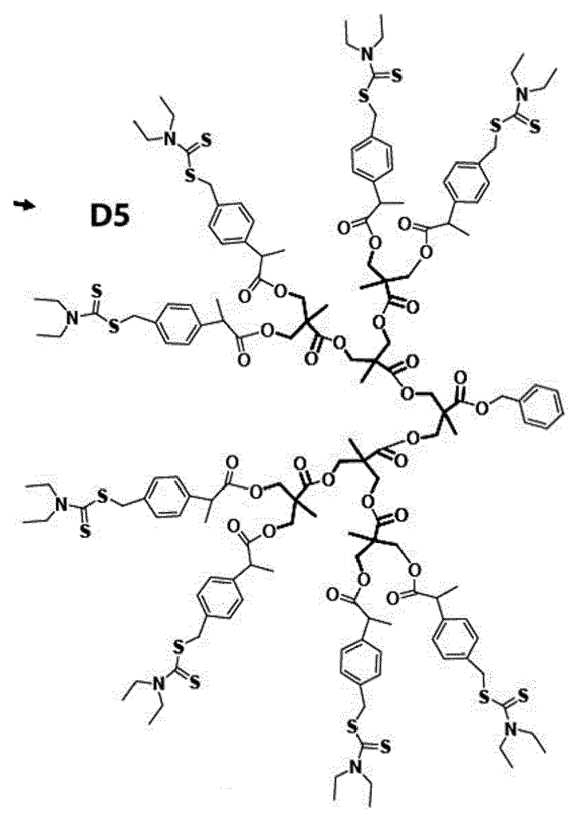 D5 and

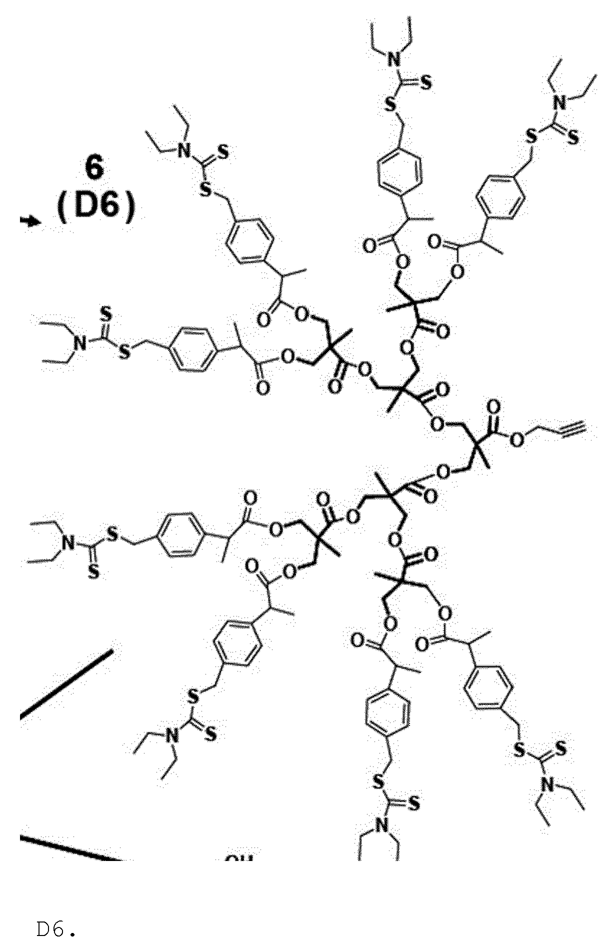

24. Use of a primer according to claim 23 for preparing a molecularly imprinted polymer through radical polymerization.
